# EUROPEAN PATENT SPECIFICATION

(11) **EP 4 112 069 B1**
(45) Date of publication and mention of the grant of the patent: **06.08.2025**
(21) Application number: 21760438.8
(22) Date of filing: 26.02.2021
(51) Int. Cl.: A61K 38/48, A61P 9/12, A61P 9/02

(54) **COMPONENTS OF THE PLASMINOGEN ACTIVATION PATHWAY FOR USE IN THE TREATMENT OF HYPERTENSION**
KOMPONENTEN DES PLASMINOGEN-AKTIVIERUNGSWEGES ZUR VERWENDUNG BEI DER BEHANDLUNG VON BLUTHOCHDRUCK
COMPOSANTS DE LA VOIE D'ACTIVATION DU PLASMINOGÈNE POUR UTILISATION DANS LE TRAITEMENT DE L'HYPERTENSION

(30) Priority: 26.02.2020 CN 202010119730
(43) Date of publication of application: 04.01.2023
(73) Proprietor: Talengen International Limited, Hong Kong 999077 (HK)
(72) Inventor: LI, Jinan, Shenzhen City Guangdong 518020 (CN)
(74) Representative: karo IP
(86) International application number: PCT/CN2021/078200
(87) International publication number: WO 2021/170099

(56) References cited:
- WO-A1-2017/101868
- WO-A1-2017/101871
- WO-A1-2018/107692
- WO-A2-02/00248
- WO-A2-2012/014214
- CN-A- 105 535 950
- CN-A- 106 890 320
- CN-A- 108 210 894
- CN-A- 108 210 895
- CN-A- 108 210 897
- CN-A- 108 210 909
- CN-A- 108 210 915
- CN-A- 108 210 917
- CN-A- 109 125 715
- LEVI M ET AL: "DEFICIENCY OF UROKINASE-TYPE PLASMINOGEN ACTIVATOR-MEDIATED PLASMIN GNERATION IMPAIRS VASCULAR REMODELING DURING HYPOXIA-INDUCED PULMONARY HYPERTENSION IN MICE", CIRCULATION, AMERICAN HEART ASSOCIATION, US, vol. 103, no. 15, 1 January 2001 (2001-01-01), pages 2014 - 2020, XP001053414, ISSN: 0009-7322
- JENG J-R ET AL: "Effect of doxazosin on fibrinolysis in hypertensive patients with and without insulin resistance", AMERICAN HEART JOURNAL, ELSEVIER, AMSTERDAM, NL, vol. 132, no. 4, 1 October 1996 (1996-10-01), pages 783 - 789, XP004530812, ISSN: 0002-8703, DOI: 10.1016/S0002-8703(96)90312-9
- SVENNINGSEN PER ET AL: "Physiology and pathophysiology of the plasminogen system in the kidney", PFLÜGERS ARCHIV - EUROPEAN JOURNAL OF PHYSIOLOGY, SPRINGER BERLIN HEIDELBERG, BERLIN/HEIDELBERG, vol. 469, no. 11, 27 June 2017 (2017-06-27), pages 1415 - 1423, XP036333037, ISSN: 0031-6768, [retrieved on 20170627], DOI: 10.1007/S00424-017-2014-Y

## Description

### TECHNICAL FIELD

The present application relates to a method and medicament for treating abnormal blood pressure condition and a complication thereof.

### BACKGROUND ART

Hypertension is one of the frequently-occurring diseases in the world, and its incidence is increasing year by year in China. Hypertension leads to complications of the disease of heart, brain, kidney or other internal organ, resulting in disability and death. The harm of hypertension is that it can lead to the lesions of multiple organs and systems such as the heart, brain, and kidney, etc., for example, the most common complication of stroke. Second, high blood pressure leads to associated cardiac damage, including myocardial hypertrophy, coronary arteriosclerosis, arrhythmias, and heart failure. Hypertension is also often accompanied by kidney damage and peripheral vascular lesions. If hypertension develops to the middle and late stages, retinopathy can occur. Diabetes is also one of the common comorbidities of hypertension. The research on prevention and treatment of hypertension has attracted more and more attention from scholars all over the world. At the same time, effective prevention and treatment of the complications of hypertension can significantly reduce the morbidity and mortality of patients.

In the present application, it is found that plasminogen can significantly reduce high blood pressure, while improving tissue and organ damage, fibrosis and dysfunction caused by hypertension, and opening up a new way for the prevention and treatment of hypertension and related diseases and complications.

### SUMMARY OF THE APPLICATION

The references to methods of treatment in this description are to be interpreted as references to the compounds, pharmaceutical compositions and medicaments of the present invention for use in a method for treatment of the human (or animal) body by therapy. The invention is defined by the appended claims.

The present application relates to a component of the plasminogen activation pathway, for example, a method, use and medicament for preventing and treating abnormal blood pressure condition (including hypertension and hypotension) by using plasminogen. The present application demonstrates through research that a component of the plasminogen activation pathway, such as plasminogen, can promote the blood pressure in a subject suffering from hypertension or hypotension to return to normal, while reducing and alleviating structural damage and functional damage of tissues and organs (such as the heart, lungs, kidneys and liver) caused by abnormal blood pressure condition (e.g., hypertension or hypotension).

Particularly, the present application relates to the following items:
In one aspect, which is not encompassed by the wording of the claims, the application relates to a method for preventing or treating hypertension, comprising: administrating to a hypertensive subject an effective amount of one or more compounds selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In one aspect, which is not encompassed by the wording of the claims, the present application further relates to use of one or more compounds in the preparation of a medicament for preventing or treating hypertension, wherein said one or more compounds are selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In one aspect, which is not encompassed by the wording of the claims, the present application further relates to a medicament for preventing or treating hypertension which comprises one or more compounds selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In one aspect, the present application relates to a pharmaceutical composition comprising an effective amount of component of plasminogen activation pathway for use in treating an abnormal blood pressure condition in a subject suffering from the abnormal blood pressure condition, wherein the abnormal blood pressure condition is hypertension, wherein the component of plasminogen activation pathway is selected from the group consisting of: plasminogen, recombinant human plasmin, Lys-plasminogen, Glu-plasminogen, plasmin, a variant or an analog of plasminogen or plasmin comprising one or more kringle domains and protease domains of plasminogen and plasmin, mini-plasminogen, mini-plasmin, micro-plasminogen, micro-plasmin, delta-plasminogen, delta-plasmin, tPA and uPA.

In one aspect, which is not encompassed by the wording of the claims, the present invention relates to a method, use or medicament according to aforementioned item, wherein the antagonist of the fibrinolysis inhibitor is an inhibitor of PAI-1, complement C1 inhibitor, α2 antiplasmin or α2 macroglobulin, e.g., an antibody.

The pharmaceutical composition as mentioned above, wherein the hypertension comprises damage of heart, brain, lung, liver, kidney, or blood vessel caused by high blood pressure or a complication thereof.

The pharmaceutical composition as above-mentioned, wherein the complications include: arrhythmia, heart failure, cerebral hemorrhage, cerebral thrombosis, cerebral infarction, hypertensive nephropathy, renal failure, uremia, liver cirrhosis, pulmonary hypertension, pulmonary fibrosis and microthrombosis.

The pharmaceutical composition as above-mentioned, wherein the compound has one or more effects selected from the group consisting of: lowering blood pressure in a hypertensive subject, reducing the level of serum angiotensin II in a hypertensive subject, adjusting the level of ACE or ACE2 in a subject, alleviating tissue and organ damage caused by hypertension, promoting repair of the damaged tissue and organ, reducing tissue and organ fibrosis, and promoting free radical scavenging.

The pharmaceutical composition of the present invention as above-mentioned, wherein said promoting repair of the damaged tissue and organ is promoting recovery of the structure or function of damaged heart tissue, brain tissue, lung tissue, kidney tissue or liver tissue; wherein said reducing tissue organ fibrosis comprises reducing fibrosis of heart tissue, lung tissue, kidney tissue or liver tissue.

The pharmaceutical composition as above-mentioned , wherein the compound eliminates free radicals by promoting SOD production.

The pharmaceutical composition as above-mentioned , wherein the compound is plasminogen.

The pharmaceutical composition as above-mentioned, wherein the plasminogen has at least 75%, 80%, 85%, 90%, 95%, 96%, 97%, 98% or 99% sequence identity with SEQ ID NO: 2, and still has the proteolytic or lysine-binding activity of plasminogen.

The pharmaceutical composition as above-mentioned, wherein the plasminogen comprises the plasminogen active fragment represented by SEQ ID NO: 14, and has the proteolytic activity of plasminogen.

The pharmaceutical composition as aforementioned, wherein the plasminogen is selected from the group consisting of: Glu-plasminogen, Lys-plasminogen, mini-plasminogen, micro-plasminogen, delta-plasminogen, or a variant thereof retaining the proteolytic activity of plasminogen; .

The pharmaceutical composition as mentioned above, wherein the plasminogen is natural or synthetic human plasminogen, or a variant or fragment thereof which retains the proteolytic or lysine-binding activity of plasminogen.

The pharmaceutical composition as above mentioned, wherein the compound is used in combination with one or more other therapeutic methods or medicaments.

The pharmaceutical composition as mentioned above, wherein the other medicaments include hypotensive drug, hormone, immunosuppressant, antibiotics, or antiviral drug.

The pharmaceutical composition as mentioned above, wherein the compound is administered by nasal inhalation, aerosol inhalation, nasal drop, ear drop or eye drop, and intravenous administration, intraperitoneal administration, subcutaneous administration, intracranial administration, intrathecal administration, intra-arterial administration, intra-rectal administration and/or intramuscular administration.

Particularly, the present application further relates to the following items:
In one aspect, which is not encompassed by the wording of the claims, the application relates to a method for preventing or treating abnormal blood pressure or abnormal blood pressure condition, comprising: administrating to a subject suffering from abnormal blood pressure condition an effective amount of one or more compounds selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In one aspect, which is not encompassed by the wording of the claims, the present application further relates to use of one or more compounds in the preparation of a medicament for preventing or treating abnormal blood pressure or abnormal blood pressure condition, wherein said one or more compounds are selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In one aspect, which is not encompassed by the wording of the claims, the present application further relates to a medicament for preventing or treating abnormal blood pressure or abnormal blood pressure condition which comprises one or more compounds selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In one aspect, which is not encompassed by the wording of the claims, the present application relates to use of one or more compounds in the prevention and treatment of abnormal blood pressure or abnormal blood pressure condition, said one or more compounds are selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

Abnormal blood pressure or abnormal blood pressure condition described herein include high blood pressure or hypertension in which the blood pressure is higher than normal, and low blood pressure or hypotension in which the blood pressure is lower than normal. Accordingly, the present application relates to a method, use and medicament for returning the high blood pressure in a hypertensive subject or the low blood pressure in a hypotensive subject to normal level by using one or more of the compounds described above.

In one aspect, which is not encompassed by the wording of the claims, the method, use or medicament according to item mentioned above, wherein the component of plasminogen activation pathway is selected from the group consisting of: plasminogen, recombinant human plasmin, Lys-plasminogen, Glu-plasminogen, plasmin, a variant or an analog of plasminogen or plasmin comprising one or more kringle domains and protease domains of plasminogen and plasmin, mini-plasminogen, mini-plasmin, micro-plasminogen, micro-plasmin, delta-plasminogen, delta-plasmin, an activator of plasminogen, tPA and uPA.

In one aspect, which is not encompassed by the wording of the claims, the method, use or medicament according to item mentioned above, wherein the antagonist of the fibrinolysis inhibitor is an inhibitor of PAI-1, complement C1 inhibitor, α2 antiplasmin or α2 macroglobulin, e.g., an antibody.

In one aspect, which is not encompassed by the wording of the claims, the method, use or medicament according to any one of items mentioned above, wherein the abnormal blood pressure condition comprises tissue and organ damage or a complication thereof caused by the abnormal blood pressure condition. In some embodiments, the tissue and organ damage or a complication thereof is a damage of the heart, brain, lung, liver, kidney or blood vessel or a complication thereof. In some embodiments, the tissue and organ damage is structural damage of tissue and organ (e.g., a change of normal tissue structure) or function damage of tissue and organ (e.g., a decline of tissue and organ function).

In the above embodiments, the abnormal blood pressure condition comprises hypertension or hypotension.

In one aspect, which is not encompassed by the wording of the claims, the method, use or medicament according to item 4, wherein the complication caused by the abnormal blood pressure condition is a complication caused by hypertension, including arrhythmia, heart failure, coronary heart disease, cerebral hemorrhage, cerebral thrombosis, cerebral infarction, hypertensive nephropathy, renal failure, uremia, liver cirrhosis, pulmonary hypertension, pulmonary fibrosis or microthrombosis.

The method, use or medicament according to above-mentioned item, wherein the complication caused by the abnormal blood pressure condition is a complication caused by hypotension, including blood-supply insufficiency to tissue and organ caused by hypotension, such as blood-supply insufficiency to heart, angina pectoris, shock, blood-supply insufficiency to brain, syncope, cerebral infarction, blood-supply insufficiency to kidney, oliguria, proteinuria, renal insufficiency.

In one aspect, which is not encompassed by the wording of the claims, the method, use or medicament according to any one of items mentioned above, wherein the compound has one or more effects selected from the group consisting of: lowering blood pressure in a hypertensive subject, reducing the level of serum angiotensin II in a hypertensive subject, adjusting the level of ACE or ACE2 in a subject, alleviating tissue and organ damage caused by hypertension, promoting repair of the damaged tissue and organ, reducing tissue and organ fibrosis, and promoting free radical scavenging.

In one aspect, which is not encompassed by the wording of the claims, the method, use or medicament according to any one of items mentioned above, wherein said promoting repair of the damaged tissue and organ is promoting recovery of the structure or function of damaged heart tissue, brain tissue, lung tissue, kidney tissue or liver tissue;
wherein said reducing tissue organ fibrosis comprises reducing fibrosis of heart tissue, lung tissue, kidney tissue or liver tissue;
wherein the compound eliminates free radicals by promoting SOD production;
wherein the compound is plasminogen.

In one aspect, which is not encompassed by the wording of the claims, the method, use or medicament according to any one of items mentioned above, wherein the plasminogen has at least 75%, 80%, 85%, 90%, 95%, 96%, 97%, 98% or 99% sequence identity with SEQ ID NO: 2, and still has the proteolytic or lysine-binding activity of plasminogen.

In another aspect, which is not encompassed by the wording of the claims, wherein the plasminogen comprises the plasminogen active fragment represented by SEQ ID NO: 14, and has the proteolytic activity of plasminogen.

In one aspect, which is not encompassed by the wording of the claims, the method, use or medicament according to any one of items mentioned above, wherein the plasminogen is selected from the group consisting of: Glu-plasminogen, Lys-plasminogen, mini-plasminogen, micro-plasminogen, delta-plasminogen, or a variant thereof retaining the proteolytic activity of plasminogen.

In another aspect, which is not encompassed by the wording of the claims, wherein the plasminogen is natural or synthetic human plasminogen, or a variant or fragment thereof which retains the proteolytic or lysine-binding activity of plasminogen.

In another aspect, which is not encompassed by the wording of the claims, wherein the compound is used in combination with one or more other therapeutic methods or medicaments.

In another aspect, which is not encompassed by the wording of the claims, wherein the other medicaments include hypotensive drug, hormone, immunosuppressant, antibiotics, or antiviral drug.

In another aspect, which is not encompassed by the wording of the claims, wherein the compound is administered by nasal inhalation, aerosol inhalation, nasal drop, ear drop or eye drop, intravenous administration, intraperitoneal administration, subcutaneous administration, intracranial administration, intrathecal administration, intra-arterial administration, intra-rectal administration and/or intramuscular administration.

In one aspect, the present application also relates to plasminogen, medicament comprising plasminogen, pharmaceutical composition, kit, or product for preventing or treating abnormal blood pressure condition (e.g., hypertension or hypotension) and the related damages or complications in a subject.

In one aspect, the present application also relates to plasminogen, medicament comprising plasminogen, pharmaceutical composition, kit, or product; use of the medicament, pharmaceutical composition, kit, or product according to the above methods in the prevention or treatment of abnormal blood pressure condition (e.g., hypertension or hypotension) and the related damages or complications in a subject.

In any of the above embodiments of the application, the plasminogen may have at least 75%, 80%, 85%, 90%, 95%, 96%, 97%, 98% or 99% sequence identity with SEQ ID NO: 2, 6, 8, 10 or 12, and still have plasminogen activity. In some embodiments, the plasminogen is a protein with addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1 -3, 1-2, or 1 amino acid based on SEQ ID NO: 2, 6, 8, 10 or 12, and still has proteolytic activity or lysine binding activity.

In some embodiments, the plasminogen is a protein comprising a fragment with proteolytic activity or lysine binding activity and still having proteolytic activity or lysine binding activity. In some embodiments, the plasminogen is selected from the group consisting of: Glu-plasminogen, Lys-plasminogen, mini-plasminogen, micro-plasminogen, delta-plasminogen, or variants thereof retaining proteolytic activity or lysine binding activity. In some embodiments, the plasminogen is natural or synthetic human plasminogen, or a variant or fragment thereof still retaining proteolytic activity or lysine binding activity. In some embodiments, the plasminogen is a human plasminogen ortholog from a primate or rodent, or a variant or fragment thereof still retaining proteolytic activity or lysine binding activity. In some embodiments, the amino acid sequence of the plasminogen is represented by SEQ ID NO: 2, 6, 8, 10 or 12. In some embodiments, the plasminogen is human natural plasminogen.

In some embodiments, the subject is a human. In some embodiments, the subject is deficient or lacking in plasminogen. In some embodiments, the lack or deficiency of plasminogen is congenital, secondary and/or local.

In some embodiments, the pharmaceutical composition comprises a pharmaceutically acceptable carrier and plasminogen for use in the above methods. In some embodiments, the kit may be a prophylactic or therapeutic kit, comprising: (i) plasminogen for use in the above methods, and (ii) means for delivering the plasminogen to the subject. In some embodiments, the means is a syringe or vial. In some embodiments, the kit further comprises a label or instructions for administrating the plasminogen to the subject to perform any of the above methods.

In some embodiments, the product comprises: a container comprising a label; and further comprises (i) plasminogen for use in the above method or a pharmaceutical composition comprising plasminogen, wherein the label instructs the administration of the plasminogen or composition to the subject to perform any of the above methods.

In some embodiments, the kit or product further comprises one or more additional means or containers containing other medicaments.

In some embodiments of the above methods, the plasminogen is administrated by systemic or topical administration for therapy, preferably by the following routes: nasal inhalation, aerosol inhalation, nasal drop or eye drop; and intravenous administration, intraperitoneal administration, subcutaneous administration, intracranial administration, intrathecal administration, intra-arterial administration, intra-rectal administration, or intramuscular administration of plasminogen. In some embodiments of the above methods, the plasminogen is administrated in combination with a suitable polypeptide carrier or a stabilizer. In some embodiments of the above methods, the plasminogen is administrated per day at the amount of 0.0001-2000 mg/kg, 0.001-800 mg/kg, 0.01-600 mg/kg, 0.1-400 mg/kg, 1-200 mg/kg, 1-100 mg/kg, or 10-100mg/kg (by per kilogram of body weight); or 0.0001-2000 mg/cm², 0.001-800 mg/cm², 0.01-600 mg/cm², 0.1-400 mg/cm², 1-200 mg/cm², 1-100 mg/cm², or 10-100 mg/cm² (by per square centimeter of body surface area), preferably repeating at least once, and preferably administrating at least daily.

The present application explicitly encompasses all the combinations of the technical features belonging to the embodiments of the present application, and these combined technical solutions have been explicitly disclosed in this application, just as the separately and explicitly disclosed above technical solutions. In addition, the present application also explicitly encompasses the combinations of each embodiment and its elements, and the combined technical solutions are explicitly disclosed herein.

### BRIEF DESCRIPTION OF THE DRAWINGS

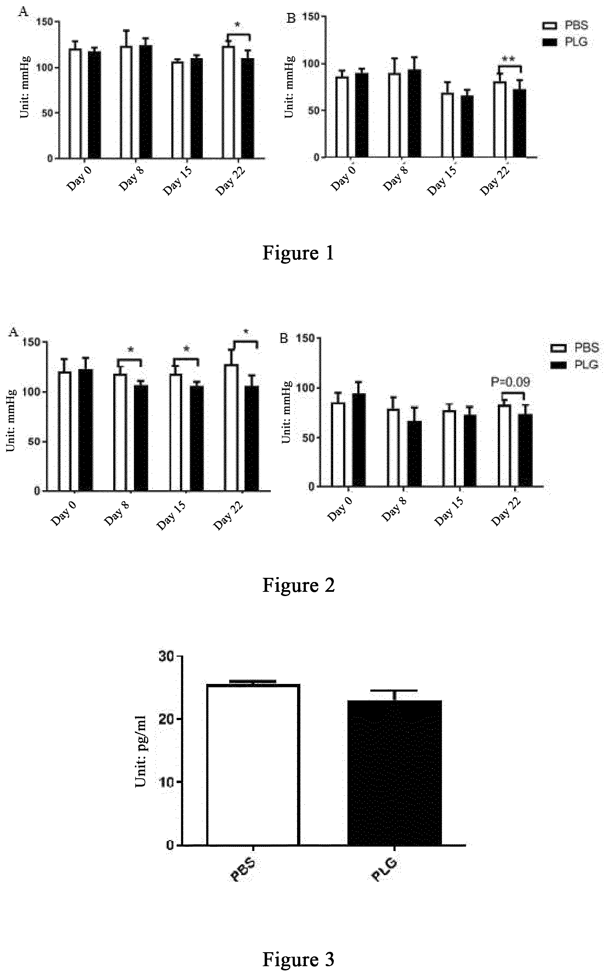

Figs. 1A-1B show the blood pressure detection results of 15-16 weeks old model mice of hypertension after administering plasminogen for 21 days. Fig. 1A shows systolic blood pressure, and Fig. 1B shows mean blood pressure. The results show that after administering plasminogen for 21 days, the blood pressure of the mice in the group in which the mice are given the vehicle PBS (*hereinafter referred to as* vehicle PBS control group, PBS control group, or vehicle group) does not change significantly as compared with that before administration, while the systolic blood pressure and mean blood pressure of the mice in the group in which the mice are given plasminogen (*hereinafter referred to as* plasminogen group) are significantly lower than those of the mice in the PBS control group; and the statistical difference is significant (* means P<0.05, ** means P<0.01), indicating that plasminogen can significantly reduce hypertension in 15-16 weeks old diabetic mice.
Figs. 2A-2B show the blood pressure detection results of 25-26 weeks old model mice of hypertension after administering plasminogen for 21 days. Fig. 2A shows systolic blood pressure, and Fig. 2B shows mean blood pressure. The results show that after administering plasminogen for 21 days, the systolic blood pressure of the mice in the PBS control group does not change significantly as compared with that before administration, while the systolic blood pressure of the mice in the plasminogen group begins to decrease significantly after administering plasminogen for 7 days, and is significantly lower than that of the mice in the vehicle PBS control group; and the statistical difference is significant (P=0.019); after administering plasminogen for 14 days and 21 days, the difference between the two groups of mice is also significant. After administering plasminogen for 21 days, the mean blood pressure of the mice in the plasminogen group is lower than that in the vehicle PBS control group, and the difference is close to significant (P=0.09), indicating that plasminogen can significantly reduce the high systolic blood pressure in 25-26 weeks old diabetic mice.
Fig. 3 shows the detection results of serum angiotensin II in 15-16 weeks old model mice of diabetic hypertension after administering plasminogen for 28 days. The results show that after administering plasminogen for 28 days, the serum angiotensin II level in the plasminogen group is significantly lower than that in the vehicle PBS control group, indicating that plasminogen can reduce the level of serum angiotensin II in the model mice of diabetic hypertension, thereby improving hypertension.

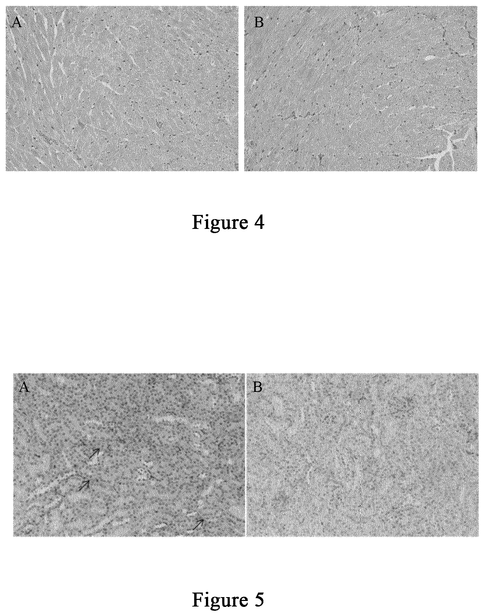

Figs. 4A-4B show representative images of cardiac H&E staining in 25-26 weeks old model mice of diabetic hypertension after administering plasminogen for 28 days. Fig. 4A is a vehicle PBS control group, and Fig. 4B is the plasminogen group. The results show that, compared with the vehicle PBS control group (Fig. 4A), the cardiomyocytes in the mice of the plasminogen group (Fig. 4B) are more compact and arranged more regularly.
Figs. 5A-5B show representative images of renal SR staining in 25-26 weeks old model mice of diabetic hypertension after administering plasminogen for 28 days. Fig. 5A is the vehicle PBS control group, and Fig. 5B is the plasminogen group. The results show that, after administering plasminogen for 28 days, the deposition of collagen fibers (marked by arrows) in the kidneys of the plasminogen group is significantly less than that of the vehicle PBS control group, indicating that plasminogen can significantly reduce renal fibrosis in the model mice of diabetic hypertension.

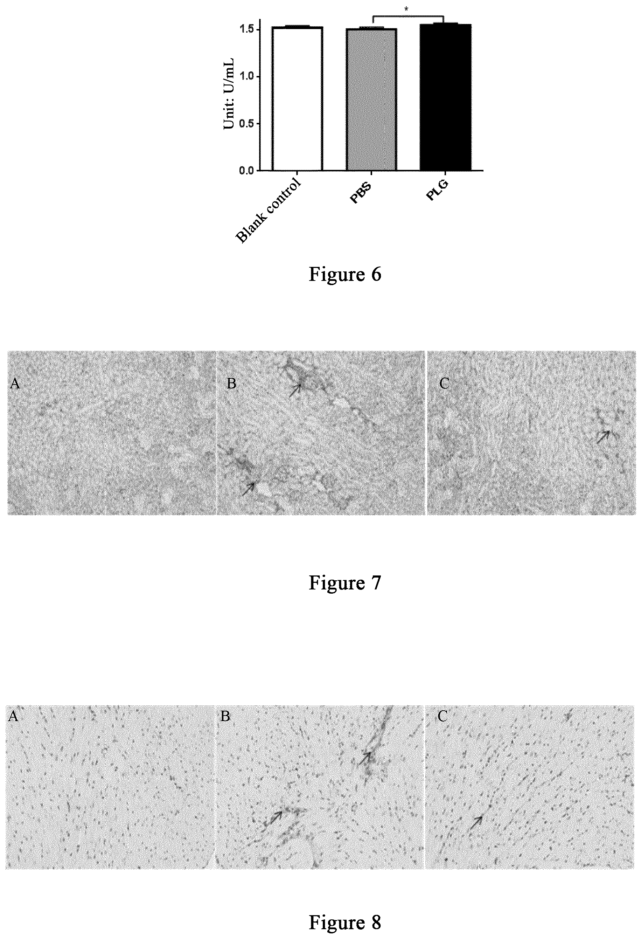

Fig. 6 shows the detection results of serum SOD level in the model mice of angiotensin II-induced hypertension after administering plasminogen for 14 days. The results show that, there is a certain level of SOD in the serum of the blank control group, the serum SOD level of the vehicle PBS group is significantly reduced, and the serum SOD level of the plasminogen group is significantly higher than that of the vehicle PBS control group, and the statistical difference is significant (* means P<0.05), indicating that plasminogen can enhance the body's ability to scavenge free radicals.
Figs. 7A-7C show the representative images of renal Sirius red staining in the model mice of angiotensin II-induced hypertension after administrating plasminogen for 14 days. Fig. 7A is the blank control group, Fig. 7B is the vehicle PBS control group, and Fig. 7C is the plasminogen group. The results show that, there is no obvious deposition of collagen fibers in the kidneys of the mice in the blank control group, and the deposition of collagen fibers (marked by arrows) in the plasminogen group is significantly less than that in the vehicle PBS control group, indicating that plasminogen can significantly reduce the renal fibrosis in the model mice of angiotensin II-induced hypertension, thereby alleviating the renal lesions caused by hypertension.
Figs. 8A-8C show the representative images of Sirius red staining of heart in the model mice of angiotensin II-induced hypertension after administrating plasminogen for 14 days. Fig. 8A is the blank control group, Fig. 8B is the vehicle PBS control group, and Fig. 8C is the plasminogen group. The results show that, there is no obvious deposition of collagen fibers in the hearts of the mice in the blank control group, and the deposition of collagen fibers (marked by arrows) in the plasminogen group is significantly less than that in the vehicle PBS control group, indicating that plasminogen can significantly reduce the cardiac fibrosis in the model mice of angiotensin II-induced hypertension, thereby alleviating the cardiac lesions caused by hypertension.

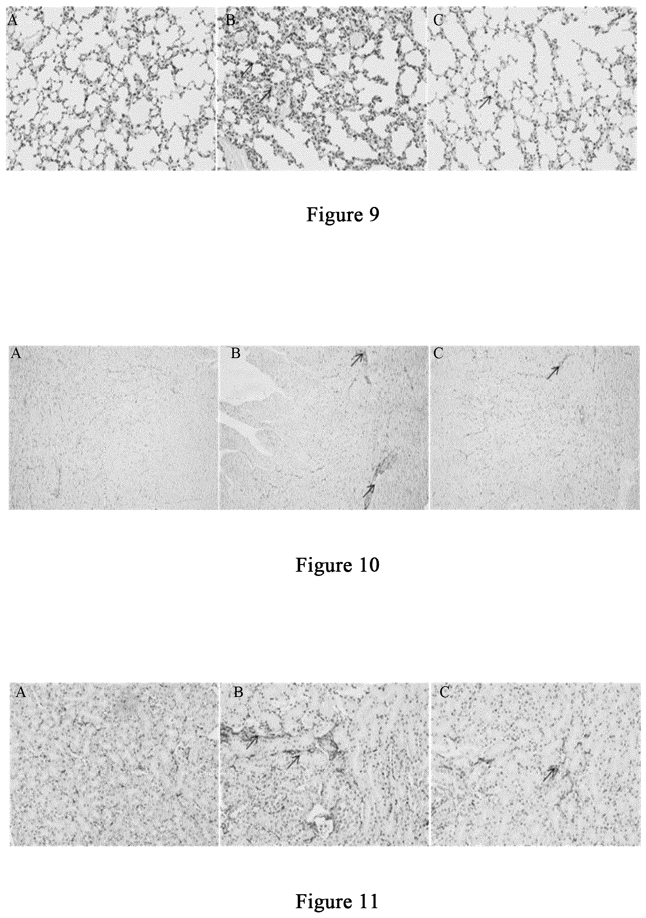

Figs. 9A-9C show the representative images of Sirius red staining of lung in the model mice of monocrotaline-induced pulmonary hypertension after administrating plasminogen for 28 days. Fig. 9A is the blank control group, Fig. 9B is the vehicle PBS control group, and Fig. 9C is the plasminogen group. The results show that, there is basically no collagen deposition in the lungs of the mice in the blank control group, and the collagen deposition (marked by arrows) in the lung tissue of the mice in the plasminogen group is significantly less than that in the vehicle PBS control group, indicating that plasminogen can significantly reduce the fibrosis of the lungs of the model mice of monocrotaline-induced pulmonary hypertension.
Figs. 10A-10C show the representative images of Sirius red staining of heart in the model mice of monocrotaline-induced pulmonary hypertension after administrating plasminogen for 28 days. Fig. 10A is the blank control group, Fig. 10B is the vehicle PBS control group, and Fig. 10C is the plasminogen group. The results show that, there is basically no collagen deposition in the heart of the mice in the blank control group, and the collagen deposition (marked by arrows) in the heart of the mice in the plasminogen group is significantly less than that in the vehicle PBS control group, indicating that plasminogen can significantly reduce the fibrosis of the heart of the model mice of monocrotaline-induced pulmonary hypertension.
Figs. 11A-11C show the representative images of Sirius red staining of kidney in the model mice of monocrotaline-induced pulmonary hypertension after administrating plasminogen for 28 days. Fig. 11A is the blank control group, Fig. 11B is the vehicle PBS control group, and Fig. 11C is the plasminogen group. The results show that, there is basically no collagen deposition in the kidney of the mice in the blank control group, and the collagen deposition (marked by arrows) in the kidney of the mice in the plasminogen group is significantly less than that in the vehicle PBS control group, indicating that plasminogen can significantly reduce the fibrosis of the kidney of the model mice of monocrotaline-induced pulmonary hypertension.

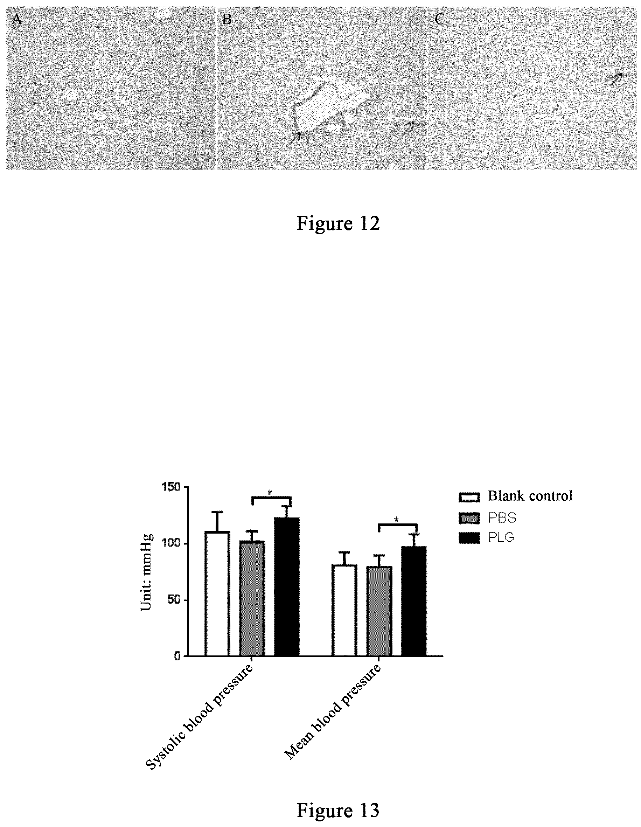

Figs. 12A-12C show the representative images of Sirius red staining of liver in the model mice of monocrotaline-induced pulmonary hypertension after administrating plasminogen for 28 days. Fig. 12A is the blank control group, Fig. 12B is the vehicle PBS control group, and Fig. 12C is the plasminogen group. The results show that, there is basically no collagen deposition in the liver of the mice in the blank control group, and the collagen deposition (marked by arrows) in the liver of the mice in the plasminogen group is significantly less than that in the vehicle PBS control group, indicating that plasminogen can significantly reduce the fibrosis of the liver of the model mice of monocrotaline-induced pulmonary hypertension.
Fig. 13 shows the detection results of blood pressure in the model mice of renal atrophy after administering plasminogen for 14 days. The results show that, the systolic blood pressure and mean blood pressure of the plasminogen group and the blank control group are significantly higher than the blood pressure of the vehicle PBS control group, and there is a statistically significant difference between the plasminogen group and the vehicle PBS control group (* means P<0.05), indicating that plasminogen can promote the hypotension caused by renal atrophy to return to normal level.

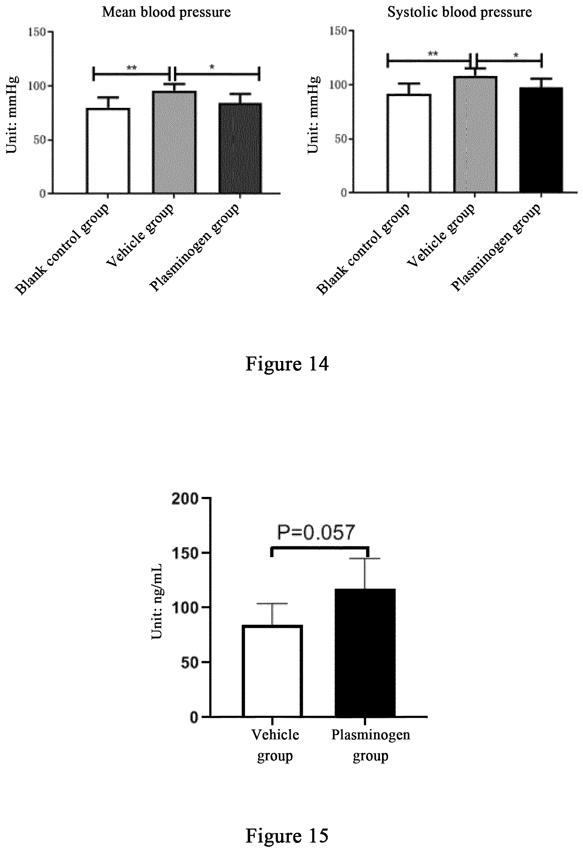

Fig. 14. shows the detection results of mean blood pressure and systolic blood pressure in the model mice of angiotensin II-induced hypertension after administering plasminogen for 5 days. The results show that, the mice in the blank control group have a certain level of mean blood pressure and systolic blood pressure, the mean blood pressure and systolic blood pressure of the mice in the vehicle group are significantly increased, and the mean blood pressure and systolic blood pressure of the mice in the plasminogen group are significantly lower than those in the vehicle group; and the statistical difference is significant (* means P<0.05, ** means P<0.01), indicating that plasminogen can reduce blood pressure in hypertension model mice.
Fig. 15 shows the detection results of serum ACE2 level in 24-25 weeks old diabetic mice after administering plasminogen for 28 days. The results show that, the serum ACE2 level of the mice in the plasminogen group is significantly higher than that in the vehicle group, and the statistical difference is close to significant (P=0.057), indicating that plasminogen can promote the increase of serum ACE2 level in the model mice of diabetic hypertension.

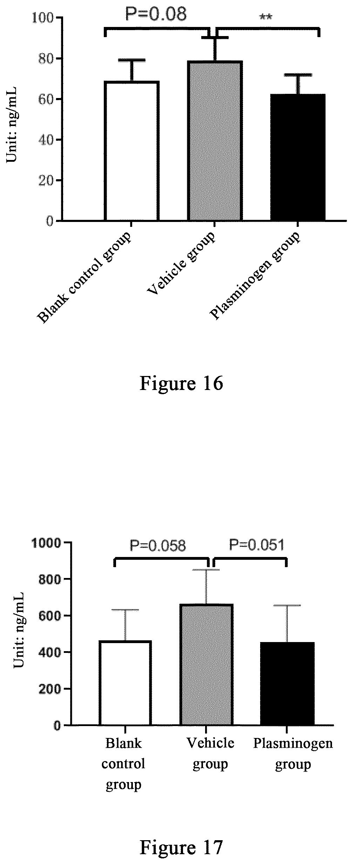

Fig. 16 shows the detection results of serum ACE2 level in the model mice of angiotensin II-induced hypertension after administering plasminogen for 7 days. The results show that, there is a certain level of ACE2 in the blood of the mice in the blank control group, the level of ACE2 in the blood of the mice in the vehicle group is significantly higher than that of the mice in the blank control group, and the level of ACE2 in the blood of the mice in the plasminogen group is significantly lower than that of the mice in the vehicle group; and the statistical difference is extremely significant (** means P<0.01), indicating that plasminogen can promote the decrease of serum ACE2 level in the model mice of angiotensin II-induced hypertension.
Fig. 17 shows the detection results of serum ACE level in the model mice of angiotensin II-induced hypertension after administering plasminogen for 7 days. The results show that, there is a certain level of ACE in the blood of the mice in the blank control group, the level of ACE in the blood of the mice in the vehicle group is significantly higher than that of the mice in the blank control group, and the level of ACE in the blood of the mice in the plasminogen group is significantly lower than that of the mice in the vehicle group; and the statistical difference is close to significant (P=0.051), indicating that plasminogen can promote the decrease of serum ACE level in the model mice of angiotensin II-induced hypertension.

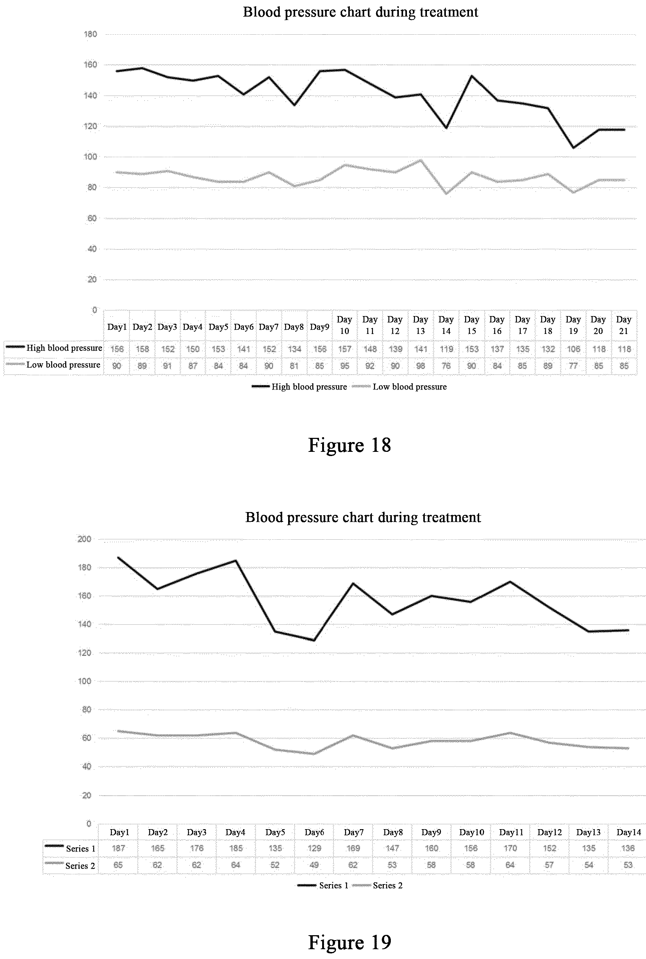

Fig. 18 shows the detection results of systolic (high pressure) and diastolic (low pressure) blood pressure during the patient's administration of plasminogen. The results show that, the patient's systolic blood pressure decreased to 141 mmHg on day 6 after the administration, then the blood pressure fluctuated unstable (alternation between normal and abnormal) until day 11; after day 12, the blood pressure decreased and remained normal and stable for a week, indicating that plasminogen can reduce blood pressure in hypertensive patients.
Fig. 19. shows the detection results of systolic (high pressure) and diastolic (low pressure) blood pressure during the patient's administration of plasminogen. The results show that, the blood pressure of the patient was 135/54 mmHg on day 13 after the administration, and was normal and stable in the later period, and basically did not need antihypertensive drugs, indicating that plasminogen can treat hypertension.

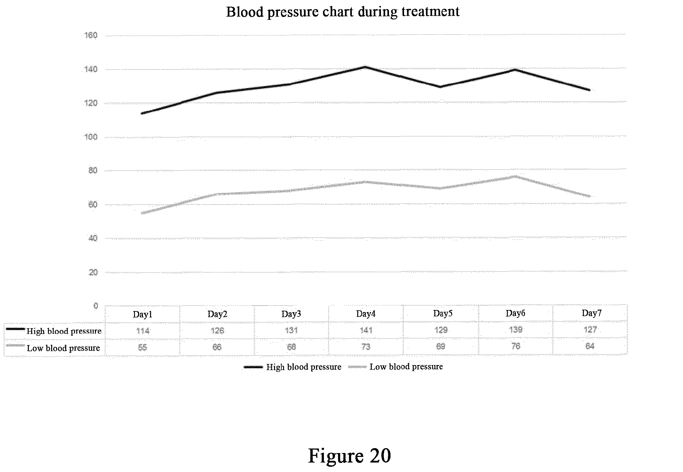

Fig. 20 shows the detection results of systolic (high pressure) and diastolic (low pressure) blood pressure during the patient's administration of plasminogen. The results show that, after the administration, the diastolic blood pressure reached the normal value, which was lower than 60mmHg before, and the blood pressure was normal and stable during the treatment; after the treatment, the antihypertensive drugs were halved, and the blood pressure was basically controlled at 130-140/64-76mmHg, indicating that plasminogen can promote blood pressure in hypertensive patients to return to normal, and can reduce the dosage of antihypertensive drugs.

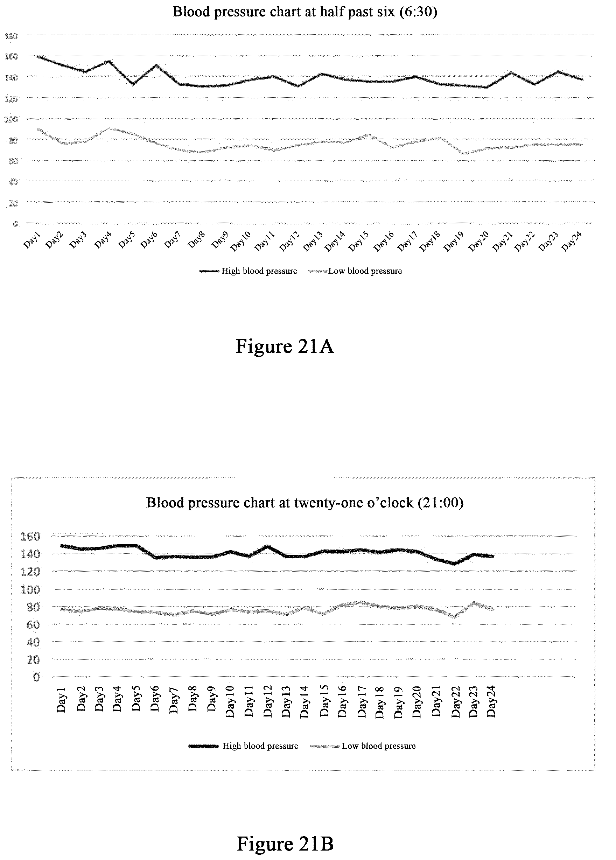

Figs. 21A-21B show the detection results of morning (Fig. 21A) and evening (Fig. 21B) systolic (high pressure) and diastolic (low pressure) blood pressure during patient's administration of plasminogen. After the administration on day 5, blood pressure began to drop in the morning and evening, and the pressure difference decreased. The patient reported that the blood pressure did not drop below 140/75mmHg while taking antihypertensive drugs before, and this was the first time it had improved. The blood pressure was maintained at about 136/73mmHg in the morning and evening after the administration on day 6. The blood pressure remained stable in the morning and evening at around 130/70 mmHg after the administration on day 7, indicating that plasminogen can alleviates the symptoms of hypertension in patients.

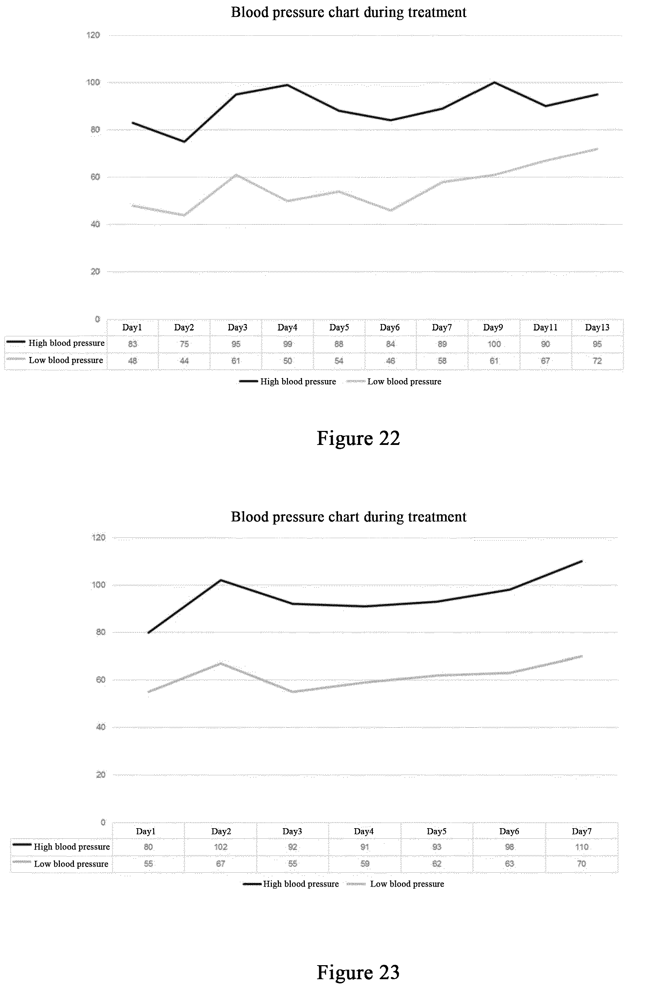

Fig. 22. shows the detection results of systolic (high pressure) and diastolic (low pressure) blood pressure during the patient's administration of plasminogen. The results show that, blood pressure showed an upward trend after the administration, and normal blood pressure appeared for the first time after administration on day 3. After day 9 of the treatment, blood pressure began to be normal and remained normal all the time, and blood pressure remained at 95/70 mmHg after drug withdrawal, indicating that plasminogen can promote blood pressure in hypotensive patients to return to normal.
Fig. 23 shows the detection results of systolic (high pressure) and diastolic (low pressure) blood pressure during the patient's administration of plasminogen. The results show that, the patient's mental state improved after the administration, and the blood pressure reached normal the day after the administration, and reached about 110/70mmHg a week later, indicating that administration of plasminogen can promote blood pressure in hypotensive patients to return to normal.

### DETAILED DESCRIPTION

Fibrinolytic system is a system consisting of a series of chemical substances involved in the process of fibrinolysis, mainly including plasminogen, plasmin, plasminogen activator, and fibrinolysis inhibitor. Plasminogen activators include tissue-type plasminogen activator (t-PA) and urokinase-type plasminogen activator (u-PA). t-PA is a serine protease that is synthesized by vascular endothelial cells. t-PA activates plasminogen, which is mainly carried out on fibrin; urokinase-type plasminogen activator (u-PA) is produced by renal tubular epithelial cells and vascular endothelial cells, and may directly activate plasminogen without the need for fibrin as a cofactor. Plasminogen (PLG) is synthesized by liver. When blood coagulates, a large amount of PLG is adsorbed on the fibrin network, and under the action of t-PA or u-PA it is activated into plasmin to promote fibrinolysis. Plasmin (PL) is a serine protease whose functions are as follows: degrading fibrin and fibrinogen; hydrolyzing various coagulation factors V, VIII, X, VII, XI, and II, etc.; converting plasminogen into plasmin; hydrolyzing complement, etc. Fibrinolysis inhibitors: including plasminogen activator inhibitor (PAI) and α2 antiplasmin (α2-AP). PAI mainly has two forms, PAI-1 and PAI-2, which may specifically bind to t-PA in a ratio of 1:1, thereby inactivating it and activating PLG at the same time. α2-AP is synthesized by liver, and binds to PL in a ratio of 1:1 to form a complex to inhibit the activity of PL; FXIII makes α2-AP covalently bound to fibrin, reducing the sensitivity of fibrin to PL. Substances that inhibit the activity of the fibrinolytic system in vivo: PAI-1, complement C1 inhibitor; α2 antiplasmin; α2 macroglobulin.

The term "component of plasminogen activation pathway" according to the present application encompasses:
1. plasminogen, Lys-plasminogen, Glu-plasminogen, micro-plasminogen, delta-plasminogen; variants or analogs thereof;
2. plasmin and a variant or analog thereof; and
3. plasminogen activators, such as tPA and uPA, and tPA or uPA variants and analogs comprising one or more domains of tPA or uPA, such as one or more kringle domains and proteolytic domains.

"Variants" of the above plasminogen, plasmin, tPA and uPA include all naturally occurring human genetic variants as well as other mammalian forms of these proteins, as well as a protein obtained by addition, deletion and/or substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid, and still retaining the activity of plasminogen, plasmin, tPA or uPA. For example, "variants" of plasminogen, plasmin, tPA and uPA include mutational variants of these proteins obtained by substitution of such as 1-100, 1-90, 1-80, 1-70, 1-60, 1-50, 1- 45, 1-40, 1-35, 1-30, 1-25, 1-20, 1-15, 1-10, 1-5, 1-4, 1-3, 1-2, or 1 conservative amino acid.

A "plasminogen variant" of the application encompasses a protein having at least 75%, 80%, 85%, 90%, 95%, 96%, 97%, 98% or 99% sequence identity with SEQ ID NO: 2, 6, 8, 10 or 12, and still retaining proteolytic or lysine-binding activity. For example, a "plasminogen variant" according to the present application may be a protein obtained by addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid on the basis of SEQ ID NO: 2, 6, 8, 10 or 12, and still retaining proteolytic or lysine-binding activity. Particularly, the plasminogen variants according to the present application include all naturally occurring human genetic variants as well as other mammalian forms of these proteins, as well as mutational variants of these proteins obtained by substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 conservative amino acid.

The plasminogen according to the present application may be a human plasminogen ortholog from a primate or rodent, or a variant thereof still retaining proteolytic or lysine-binding activity, for example, a plasminogen represented by SEQ ID NO: 2, 6, 8, 10 or 12, such as a human natural plasminogen represented by SEQ ID NO: 2.

The "analogs" of the above plasminogen, plasmin, tPA, and uPA include compounds that respectively provide substantially similar effect to plasminogen, plasmin, tPA, or uPA.

The "variants" and "analogs" of above plasminogen, plasmin, tPA and uPA encompass "variants" and "analogs" of plasminogen, plasmin, tPA and uPA comprising one or more domains (e.g., one or more kringle domains and proteolytic domains). For example, "variants" and "analogs" of plasminogen encompass "variants" and "analogs" of plasminogen comprising one or more plasminogen domains (e.g., one or more kringle domains and proteolytic domains), such as mini-plasminogen. "Variants" and "analogs" of plasmin encompass "variants" and "analogs" of plasmin comprising one or more plasmin domains (e.g., one or more kringle domains and proteolytic domains), such as mini-plasmin, and delta-plasmin.

Whether a "variant" or "analog" of the above plasminogen, plasmin, tPA or uPA respectively has the activity of plasminogen, plasmin, tPA or uPA, or whether the "variant" or "analog" provides substantially similar effect to plasminogen, plasmin, tPA or uPA, may be detected by methods known in the art, for example, it is measured by the level of activated plasmin activity based on enzymography, ELISA (enzyme-linked immunosorbent assay), and FACS (fluorescence-activated cell sorting method), for example, it is detected by referring to a method selected from the following documents: Ny, A., Leonardsson, G., Hagglund, A.C, Hagglof, P., Ploplis, V.A., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest.74(5):1625-33; Gravanis I, Tsirka SE (February 2008). "Tissue-type plasminogen activator as a therapeutic target in stroke". Expert Opinion on Therapeutic Targets. 12(2):159-70; Geiger M, Huber K, Wojta J, Stingl L, Espana F, Griffin JH, Binder BR (Aug 1989). "Complex formation between urokinase and plasma protein C inhibitor in vitro and in vivo". Blood.74(2):722-8.

In some embodiments of the present application, the "component of plasminogen activation pathway" according to the present application is a plasminogen selected from the group consisting of: Glu-plasminogen, Lys-plasminogen, mini-plasminogen, micro-plasminogen, delta-plasminogen, or variants thereof retaining proteolytic or lysine-binding activity. In some embodiments, the plasminogen is natural or synthetic human plasminogen, or a conservative mutant variant or fragment thereof still retaining proteolytic or lysine-binding activity. In some embodiments, the plasminogen is a human plasminogen ortholog from a primate or rodent or a conservative mutant variant or fragment thereof still retaining proteolytic or lysine-binding activity. In some embodiments, the amino acid sequence of the plasminogen is represented by SEQ ID NO: 2, 6, 8, 10 or 12. In some embodiments, the plasminogen is a human natural plasminogen. In some embodiments, the plasminogen is a human natural plasminogen represented by SEQ ID NO: 2.

"A compound capable of directly activating plasminogen, or indirectly activating plasminogen by activating an upstream component of plasminogen activation pathway", refers to any compound capable of directly activating plasminogen, or indirectly activating plasminogen by activating an upstream component of plasminogen activation pathway, such as tPA, uPA, streptokinase, saruplase, alteplase, reteplase, tenecteplase, anistreplase, monteplase, lanoteplase, pamiteplase, staphylokinase.

The "antagonist of a fibrinolysis inhibitor" according to the present application is a compound that antagonizes, weakens, blocks, or prevents the action of a fibrinolysis inhibitor. Such fibrinolysis inhibitors are e.g., PAI-1, complement C1 inhibitor, α2 antiplasmin, and α2 macroglobulin. Such an antagonist is: e.g., an antibody of PAI-1, complement C1 inhibitor, α2 antiplasmin, or α2 macroglobulin; or an antisense RNA or small RNA blocking or downregulating the expression of such as PAI-1, complement C1 inhibitor, α2 antiplasmin or α2 macroglobulin; or a compound occupying the binding site of PAI-1, complement C1 inhibitor, α2 antiplasmin, or α2 macroglobulin but without the function of PAI-1, complement C1 inhibitor, α2 antiplasmin, or α2 macroglobulin; or a compound blocking the binding domains and/or active domains of PAI-1, complement C1 inhibitor, α2 antiplasmin, or α2 macroglobulin.

Plasmin is a key component of the plasminogen activation system (PA system). It is a broad-spectrum protease capable of hydrolyzing several components of the extracellular matrix (ECM), including fibrin, gelatin, fibronectin, laminin, and proteoglycans. In addition, plasmin may activate some metalloproteinase precursors (pro-MMPs) to form active metalloproteinases (MMPs). Therefore, plasmin is considered to be an important upstream regulator of extracellular proteolysis. Plasmin is formed by proteolysis of plasminogen by two physiological PAs: tissue-type plasminogen activator (tPA) or urokinase-type plasminogen activator (uPA). Due to the relatively high levels of plasminogen in plasma and other body fluids, it has traditionally been thought that the regulation of the PA system is mainly achieved through the synthesis and activity levels of PAs. The synthesis of components of PA system is strictly regulated by different factors, such as hormone, growth factor and cytokine. In addition, there are specific physiological inhibitors of plasmin and PAs. The main inhibitor of plasmin is α2-antiplasmin. The activity of PAs is inhibited by plasminogen activator inhibitor-1 (PAI-1) of both uPA and tPA, and regulated by plasminogen activator inhibitor-2 (PAI-2) which mainly inhibits uPA. Certain cell surfaces have uPA-specific cell surface receptors (uPARs) with direct hydrolytic activity.

Plasminogen is a single-chain glycoprotein consisting of 791 amino acids with a molecular weight of approximately 92 kDa. Plasminogen is mainly synthesized in liver, and is abundantly present in the extracellular fluid. The content of plasminogen in plasma is approximately 2 µM. Plasminogen is thus a huge potential source of proteolytic activity in tissues and body fluids. Plasminogen exists in two molecular forms: glutamate-plasminogen (Glu-plasminogen) and lysine-plasminogen (Lys-plasminogen). The naturally secreted and uncleaved form of plasminogen has an amino-terminal (N-terminal) glutamate, and is therefore referred to as glutamate-plasminogen. However, in the presence of plasmin, glutamate-plasminogen is hydrolyzed at Lys76-Lys77 into lysine-plasminogen. Compared with glutamate-plasminogen, lysine-plasminogen has a higher affinity for fibrin, and may be activated by PAs at a higher rate. The Arg560-Val561 peptide bond of these two forms of plasminogen may be cleaved by either uPA or tPA, resulting in the formation of a two-chain protease plasmin linked by disulfide. The amino-terminal part of plasminogen comprises five homologous tri-cycles, i.e., so-called kringles, and the carboxy-terminal part comprises the protease domain. Some kringles comprise lysine-binding sites that mediate the specific interaction of plasminogen with fibrin and its inhibitor α2-AP. A recently found plasminogen is a 38 kDa fragment, including kringles1-4, and it is a potent inhibitor of angiogenesis. This fragment is named as angiostatin, and is produced by the hydrolysis of plasminogen by several proteases.

The main substrate of plasmin is fibrin, and the dissolution of fibrin is the key to preventing pathological thrombosis. Plasmin also has substrate specificity for several components of the ECM, including laminin, fibronectin, proteoglycans, and gelatin, indicating that plasmin also plays an important role in ECM remodeling. Indirectly, plasmin may also degrade other components of the ECM, including MMP-1, MMP-2, MMP-3 and MMP-9, by converting certain protease precursors into active proteases. Therefore, it has been proposed that plasmin may be an important upstream regulator of extracellular proteolysis. In addition, plasmin has the ability to activate certain latent forms of growth factors. In vitro, plasmin also hydrolyzes components of the complement system, and releases chemotactic complement fragments.

"Plasmin" is a very important enzyme present in blood that hydrolyzes fibrin clots into fibrin degradation products and D-dimers.

"Plasminogen" is the zymogen form of plasmin. According to the sequence in swiss prot, it consists of 810 amino acids calculated by the natural human plasminogen amino acid sequence (SEQ ID NO: 4) containing the signal peptide, and the molecular weight is about 90kD, and it is a glycoprotein mainly synthesized in liver and capable of circulating in blood, the cDNA sequence encoding this amino acid sequence is represented by SEQ ID NO: 3. Full-length plasminogen contains seven domains: a C-terminal serine protease domain, an N-terminal Pan Apple (PAp) domain, and five Kringle domains (Kringle1-5). Referring to the sequence in swiss prot, its signal peptide comprises residues Met1-Gly19, PAp comprises residues Glu20-Val98, Kringle1 comprises residues Cys103-Cys181, Kringle2 comprises residues Glu184-Cys262, Kringle3 comprises residues Cys275-Cys352, Kringle4 comprises residues Cys377-Cys454, and Kringle5 comprises residues Cys481-Cys560. According to NCBI data, the serine protease domain comprises residues Val581-Arg804.

Glu-plasminogen is a human natural full-length plasminogen, consisting of 791 amino acids (without a signal peptide of 19 amino acids); the cDNA sequence encoding this amino acid sequence is represented by SEQ ID NO: 1, and the amino acid sequence is represented by SEQ ID NO: 2. In vivo, there is also a Lys-plasminogen produced by the hydrolysis of the peptide bond between amino acids 76 and 77 of Glu-plasminogen, as represented by SEQ ID NO: 6; and the cDNA sequence encoding this amino acid sequence is represented by SEQ ID NO: 5. Delta-plasminogen (δ-plasminogen) is a fragment of full-length plasminogen that lacks the Kringle2-Kringle5 structure, and only contains Kringle1 and a serine protease domain (also known as a protease domain (PD)). The amino acid sequence of delta-plasminogen (SEQ ID NO: 8) is reported in a literature, and the cDNA sequence encoding this amino acid sequence is represented by SEQ ID NO: 7. Mini-plasminogen consists of Kringle5 and a serine protease domain, and it is reported that it comprises residues Val443-Asn791 (with the Glu residue of the Glu-plasminogen sequence without the signal peptide as the starting amino acid), the amino acid sequence of the mini-plasminogen is represented by SEQ ID NO: 10, and the cDNA sequence encoding this amino acid sequence is represented by SEQ ID NO: 9. While micro-plasminogen comprises only a serine protease domain, and it is reported that its amino acid sequence comprises residues Ala543-Asn791 (with the Glu residue of the Glu-plasminogen sequence without the signal peptide as the starting amino acid); additionally, it is disclosed in patent document CN102154253A that its sequence comprises residues Lys531-Asn791 (with the Glu residue of the Glu-plasminogen sequence without the signal peptide as the starting amino acid); in the present patent application, the sequence of micro-plasminogen refers to the patent document CN102154253A, the amino acid sequence is represented by SEQ ID NO: 12, and the cDNA sequence encoding this amino acid sequence is represented by SEQ ID NO: 11.

The structure of the full-length plasminogen is also described in the article by Aisina et al. (Aisina RB, Mukhametova L I. Structure and function of plasminogen/plasmin system [J]. Russian Journal of Bioorganic Chemistry, 2014, 40(6):590-605). In this article, Aisina et al. describe that plasminogen comprises Kringle 1, 2, 3, 4, 5 domains and a serine protease domain (also called protease domain (PD))(i.e., lysine binding activity), wherein Kringles are responsible for binding of plasminogen to low or high molecular weight ligand, so that plasminogen transforms into a more open conformation that is more readily activated; the protease domain (PD) is residues Val562-Asn791; the Arg561-Val562 activating bond of plasminogen is specifically cleaved by tPA and uPA, thereby allowing plasminogen to change into plasmin; thus the protease domain (PD) is a region conferring the proteolytic activity of plasminogen.

In the present application, "plasmin" and "fibrinolytic enzyme" may be used interchangeably with the same meaning; "plasminogen" and "fibrinolytic zymogen" may be used interchangeably with the same meaning.

In the present application, "lack" of plasminogen or plasminogen activity means that the content of plasminogen in a subject is lower than that of a normal person, and is sufficiently low to affect the normal physiological function of the subject; "deficiency" of plasminogen or plasminogen activity means that the content of plasminogen in a subject is significantly lower than that of a normal person, and even the activity or expression is extremely low, and the normal physiological function may only be maintained by external supply of plasminogen.

Those skilled in the art may understand that, all technical solutions of plasminogen according to the present application are applicable to plasmin, thus the technical solutions described in the present application encompass plasminogen and plasmin. During circulation, plasminogen is present in a closed, inactive conformation, but when bound to a thrombus or cell surface, it is converted into active plasmin with an open conformation after being mediated by plasminogen activator (PA). Active plasmin may further hydrolyze the fibrin clot into degradation products of fibrin and D-dimers, thereby dissolving the thrombus. The PAp domain of plasminogen comprises an important determinant for maintaining plasminogen in an inactive closed conformation, while the KR domain may bind to a lysine residue present on a receptor and substrate. A variety of enzymes are known to act as plasminogen activators, including: tissue plasminogen activator (tPA), urokinase plasminogen activator (uPA), kallikrein, and coagulation factor XII (Hageman factor) etc.

An "active fragment of plasminogen" refers to a fragment having the activity of binding to a lysine in the target sequence of a substrate (lysine-binding activity), or exerting the activity of a proteolytic function (proteolytic activity), or having a combination of proteolytic activity and lysine-binding activity. The technical solutions related to plasminogen according to the present application encompass the technical solutions of replacing plasminogen with an active fragment of plasminogen. In some embodiments, the active fragment of plasminogen according to the present application comprises or consists of a serine protease domain of plasminogen, preferably the active fragment of plasminogen according to the present application comprises or consists of SEQ ID NO: 14, or an amino acid sequence having at least 80%, 90%, 95%, 96%, 97%, 98%, 99% identity with SEQ ID NO: 14. In some embodiments, the active fragment of plasminogen according to the present application comprises or consists of one or more regions selected from the group consisting of: Kringle 1, Kringle 2, Kringle 3, Kringle 4, and Kringle 5. In some embodiments, the plasminogen according to the present application comprises a protein comprising the active fragment of plasminogen described above.

At present, the methods for measuring plasminogen and its activity in blood comprise: detection of tissue plasminogen activator activity (t-PAA), detection of plasma tissue plasminogen activator antigen (t-PAAg), detection of plasma tissue plasminogen activity (plgA), detection of plasma tissue plasminogen antigen (plgAg), detection of the activity of plasma tissue plasminogen activator inhibitor, detection of the antigen of plasma tissue plasminogen activator inhibitor, and detection of plasma plasmin-antiplasmin complex (PAP); wherein the most commonly used detection method is the chromogenic substrate method: adding streptokinase (SK) and a chromogenic substrate to the plasma to be detected, the PLG in the plasma to be detected is converted into PLM under the action of SK, and PLM acts on the chromogenic substrate; subsequently, the detection by spectrophotometer indicates that the increase in absorbance is proportional to plasminogen activity. In addition, the plasminogen activity in blood may also be detected by immunochemical method, gel electrophoresis, immunoturbidimetry, and radioimmunoassay.

"Ortholog or orthologs" refer to homologs between different species, including both protein homologs and DNA homologs, also known as orthologs and vertical homologs; particularly it refers to proteins or genes evolved from the same ancestral gene in different species. The plasminogen according to the present application includes human natural plasminogen, and also includes plasminogen ortholog or orthologs derived from different species and having plasminogen activity.

A "conservative substitution variant" refers to a variant in which a given amino acid residue is altered without changing the overall conformation and function of the protein or enzyme, including but not limited to those variants in which the amino acid(s) in the amino acid sequence of the parent protein are replaced by amino acid(s) with similar properties (e.g., acidic, basic, hydrophobic, etc.). Amino acids with similar properties are well known in the art. For example, arginine, histidine and lysine are hydrophilic basic amino acids and are interchangeable. Similarly, isoleucine is a hydrophobic amino acid, and may be replaced by leucine, methionine or valine. Therefore, the similarity of two proteins or amino acid sequences with similar functions may differ; for example, 70% to 99% similarity (identity) based on the MEGALIGN algorithm. "Conservative substitution variants" also include polypeptides or enzymes having not less than 60%, preferably not less than 75%, more preferably not less than 85%, or even most preferably not less than 90% amino acid identity determined by BLAST or FASTA algorithm, and having the same or substantially similar properties or functions as the natural or parent protein or enzyme.

"Isolated" plasminogen refers to a plasminogen protein isolated and/or recovered from its natural environment. In some embodiments, the plasminogen will be purified: (1) to more than 90%, more than 95%, or more than 98% purity (by weight), as determined by Lowry's method, e.g., more than 99% (by weight), (2) to a degree sufficient to obtain at least 15 residues of the N-terminal or internal amino acid sequence by using a spinning cup sequence analyzer, or (3) to homogeneity as determined by using Coomassie blue or silver staining through sodium dodecyl sulfate-polyacrylamide gel electrophoresis (SDS-PAGE) under reducing or nonreducing conditions. Isolated plasminogen also includes plasminogen prepared from recombinant cells by bioengineering techniques and isolated by at least one purification step.

The terms "polypeptide", "peptide" and "protein" are used interchangeably herein to refer to a polymeric form of amino acids of any length, which may include genetically encoded and non-genetically encoded amino acids, chemically or biochemically modified or derivatized amino acids, and polypeptides with modified peptide backbones. The terms include fusion proteins including, but not limited to, fusion proteins with heterologous amino acid sequences, fusions with heterologous and homologous leader sequences (with or without N-terminal methionine residues); and the like.

"Percent (%) of amino acid sequence identity" with respect to a reference polypeptide sequence is defined as, after introducing gaps as necessary to achieve maximum percent sequence identity, and no conservative substitutions are considered as part of the sequence identity, the percentage of amino acid residues in a candidate sequence that are identical to the amino acid residues in a reference polypeptide sequence. Alignment for purposes of determining percent amino acid sequence identity may be accomplished in a variety of ways within the technical scope in the art, e.g., by publicly available computer software, such as BLAST, BLAST-2, ALIGN or Megalign (DNASTAR) software. Those skilled in the art may determine the appropriate parameters for aligning sequences, including any algorithms needed to achieve maximal alignment over the full length of the sequences to be compared. However, for the purpose of the present application, the values of percent amino acid sequence identity are generated by using the computer program ALIGN-2 for sequence comparison.

Where ALIGN-2 is used to compare amino acid sequences, the percentage (%) of amino acid sequence identity of a given amino acid sequence A relative to a given amino acid sequence B (or may be expressed as a given amino acid sequence A having a certain percentage (%) of amino acid sequence identity relative to, with or with respective to a given amino acid sequence B) is calculated as follows:
Fraction X/Y times 100;
wherein X is the number of amino acid residues scored as identical matches during the alignment of sequences A and B by the sequence alignment program ALIGN-2, and wherein Y is the total number of amino acid residues in sequence B. It should be appreciated that, where the length of amino acid sequence A is not equal to that of amino acid sequence B, the percentage (%) of amino acid sequence identity of A with respect to B will not equal to the percentage (%) of amino acid sequence identity of B with respect to A. Unless expressly stated otherwise, all the values of percentage (%) of amino acid sequence identity used herein are obtained by using the ALIGN-2 computer program as described in the preceding paragraph.

As used herein, the terms "treatment/treating" refer to obtaining a desired pharmacological and/or physiological effect. The effect may be complete or partial prevention of the occurrence, or onset of the disease or symptoms thereof, partial or complete alleviation of the disease and/or symptoms thereof, and/or partial or complete cure of the disease and/or symptoms thereof; and includes: (a) preventing the occurrence or onset of the disease in a subject, who may have predisposition of the disease, but is not yet diagnosed as having the disease; (b) inhibiting the disease, i.e., blocking its development; and (c) alleviating the disease and/or symptoms thereof, i.e., causing regression or elimination of the disease and/or symptoms thereof.

The terms "individual", "subject" and "patient" are used interchangeably herein to refer to mammals including, but not limited to, murine (rat, mouse), non-human primate, human, canine, feline, hoofed animals (e.g., horses, cattle, sheep, pigs, goats), etc.

A "therapeutically effective amount" or "effective amount" refers to an amount of a component of plasminogen activation pathway or a related compound thereof (e.g., plasminogen) sufficient to prevent and/or treat a disease when administrated to a mammal or other subject for treating the disease. A "therapeutically effective amount" will vary depending on the component of plasminogen activation pathway or a related compound thereof (e.g., plasminogen) in use, the severity of the disease and/or symptoms thereof in the subject to be treated, as well as the age, weight, and the like.

In the present application, "promoting the repair of damaged tissues and organs" refers to promoting the repair of the structure and function of damaged tissues and organs, so as to restore the integrity and function of the anatomical structure of damaged tissues and organs to normal as much as possible.

"High blood pressure" or "hypertension" herein refers to a state in which systemic arterial pressure is higher than normal. According to the Treatment Guidelines of the World Health Organization/International Society of Hypertension (1999), the diagnostic criteria for hypertension is systolic blood pressure ≥18.7Kpa (140mmHg), or diastolic blood pressure ≥12.0Kpa (90mmHg). "Low blood pressure" or "hypotension" refers to a state in which systemic arterial pressure is lower than normal. There is no uniform standard for the diagnosis of hypotension. It is generally believed that if the upper extremity arterial systolic/diastolic blood pressure in adults is respectively lower than 12/8kPa (90/60mmHg), it is regarded as hypotension. The diagnostic criteria for blood pressure indicators of "high blood pressure" or "hypertension" and "low blood pressure" or "hypotension" may also refer to the specific provisions in the diagnostic manuals of various countries.

In the present application, treating a subject with "high blood pressure" or "hypertension" includes "lowering" or "decreasing" the high blood pressure of a subject suffering from "high blood pressure" or "hypertension", the "lowering" or "decreasing" refers to a decrease in the blood pressure of a subject compared to a control without administration, or compared to the blood pressure of the subject prior to administration, e.g., making the blood pressure of the subject towards normal, near normal, or return to normal level. Likewise, in this application, treating a subject with "low blood pressure" or "hypotension" includes "elevating" the low blood pressure in a subject suffering from "low blood pressure" or "hypotension." The "elevating" means to increase the blood pressure of the subject comparing to a control without administration, or comparing to the blood pressure of the subject prior to administration, e.g., making the blood pressure of the subject towards normal, near normal, or return to normal level.

"Complication" means that one disease causes the occurrence of another disease or symptom during the development process, the latter being a complication of the former; that is, the occurrence of the latter disease is caused by the former disease, or the patient develops another or several diseases related to the disease during the diagnosis and treatment of the disease.

"Hypertensive complications" means complications caused by high blood pressure, including heart, brain, lung, liver, kidney or blood vessel damage caused by high blood pressure. Complications of heart damage caused by hypertension, such as left ventricular hypertrophy, angina pectoris, myocardial infarction and heart failure; complications of brain tissue damage caused by hypertension, such as cerebral hemorrhage, cerebral thrombosis, cerebral infarction, hemorrhagic stroke, ischemic stroke, hypertensive encephalopathy; renal damage caused by hypertension, such as slowly progressive small arterial nephrosclerosis, malignant small arterial nephrosclerosis, chronic renal failure, hypertensive nephropathy, renal failure, uremia. The most common complication of hypertension is cerebrovascular accident, followed by hypertensive heart disease, heart failure, and renal failure. A rare but serious complication is aortic dissective aneurysm.

"Hypotensive complications" refers to complications caused by low blood pressure. The common complications of hypotension are mostly caused by insufficient perfusion of vital organs. If the blood supply to the brain is insufficient, it is mostly manifested as tinnitus, dizziness, etc. In severe cases, cerebral infarction may be complicated; if the blood supply to the heart is insufficient, it is mostly manifested as palpitation, shortness of breath, chest tightness, etc.; if the blood supply to the kidney is insufficient, it is mostly manifested as oliguria, proteinuria, severe cases of renal insufficiency such as anuria.

### Preparation of the Plasminogen According to the Present Application

Plasminogen may be isolated from nature, and purified for further therapeutic use, or it may be synthesized by standard chemical peptide synthesis techniques. When the polypeptide is synthesized chemically, the synthesis may be carried out via liquid phase or solid phase. Solid-phase polypeptide synthesis (SPPS) (in which the C-terminal amino acid of the sequence is attached to an insoluble support, followed by the sequential addition of the retaining amino acids in the sequence) is a suitable method for chemical synthesis of plasminogen. Various forms of SPPS, such as Fmoc and Boc, may be used to synthesize plasminogen. Techniques for solid-phase synthesis are described in Barany and Solid-Phase Peptide Synthesis; pp.3-284 in The Peptides: Analysis, Synthesis, Biology. Vol. 2: Special Methods in Peptide Synthesis, Part A., Merrifield, et al. J. Am. Chem. Soc., 85:2149-2156 (1963); Stewart et al., Solid Phase Peptide Synthesis, 2nd ed. Pierce Chem. Co., Rockford, Ill. (1984); and Ganesan A. 2006 Mini Rev. Med Chem. 6:3-10, and Camarero JA et al. 2005, Protein Pept Lett. 12:723-8. Briefly, small insoluble porous beads are treated with functional units on which peptide chains are constructed; after repeated cycles of coupling/deprotection, the attached solid-phase free N-terminal amine is coupled to a single N-protected amino acid unit. This unit is then deprotected to reveal new N-terminal amines that may be attached to other amino acids. The peptide remains immobilized on the solid phase, subsequently it is cleaved off.

Plasminogen according to the present application may be produced by standard recombinant methods. For example, a nucleic acid encoding plasminogen is inserted into an expression vector to be operably linked to regulatory sequences in the expression vector. The regulatory sequences for expression include, but are not limited to, promoters (e.g., naturally associated or heterologous promoters), signal sequences, enhancer elements, and transcription termination sequences. Expression regulation may be a eukaryotic promoter system in a vector capable of transforming or transfecting a eukaryotic host cell (e.g., COS or CHO cell). Once the vector is incorporated into a suitable host, the host is maintained under conditions suitable for high-level expression of the nucleotide sequence and collection and purification of plasminogen.

A suitable expression vector is typically replicated in a host organism as an episome or as an integrated part of the host chromosomal DNA. Typically, an expression vector contains a selectable marker (e.g., ampicillin resistance, hygromycin resistance, tetracycline resistance, kanamycin resistance, or neomycin resistance marker) to facilitate the detection of those cells transformed with desired exogenous DNA sequence.

*Escherichia coli* is an example of a prokaryotic host cell that may be used to clone a subject compound-encoding polynucleotide. Other microbial hosts suitable for use include bacilli such as *Bacillus subtilis,* and other *enterobacteriaceae* such as *Salmonella, Serratia,* and various *Pseudomonas* species. In these prokaryotic hosts, expression vectors may also be generated, which will typically contain an expression control sequence (e.g., origin of replication) that are compatible with the host cell. In addition, there are many well-known promoters, such as the lactose promoter system, the tryptophan (trp) promoter system, the beta-lactamase promoter system, or the promoter system from bacteriophage lambda. A promoter will typically control the expression, optionally in case of an operator gene sequence, and have ribosome binding site sequence, etc., to initiate and complete transcription and translation.

Other microorganisms, such as yeast, may also be used for expression. Yeast (e.g., S. *cerevisiae)* and *Pichia* are examples of suitable yeast host cells, and as required a suitable vector has an expression control sequence (e.g., promoter), origin of replication, termination sequence, etc. A typical promoter comprises 3-phosphoglycerate kinase and other saccharolytic enzymes. Particularly, inducible yeast promoters include promoters from ethanol dehydrogenase, isocytochrome C, and enzymes responsible for maltose and galactose utilization.

In addition to microorganisms, mammalian cells (e.g., mammalian cells grown in in vitro cell culture) may also be used to express and produce the plasminogen of the application (e.g., polynucleotides encoding plasminogen). See Winnacker, From Genes to Clones, VCH Publishers, N.Y., N.Y. (1987). Suitable mammalian host cells include CHO cell lines, various Cos cell lines, HeLa cells, myeloma cell lines, and transformed B cells or hybridomas. Expression vectors for use in these cells may comprise expression control sequences such as origin of replication, promoter and enhancer (Queen et al., Immunol. Rev. 89:49 (1986)), and necessary sites for processing information such as ribosome binding sites, RNA splicing sites, polyadenylation sites, and transcription terminator sequences. Examples of suitable expression control sequences are promoters derived from immunoglobulin gene, SV40, adenovirus, bovine papilloma virus, cytomegalovirus, and the like. See Co et al, J. Immunol. 148:1149 (1992).

Once synthesized (chemically or recombinantly), the plasminogen of the present application may be purified according to standard procedures in the art, including ammonium sulfate precipitation, affinity column, column chromatography, high performance liquid chromatography (HPLC), gel electrophoresis, and the like. The plasminogen is substantially pure, e.g., at least about 80-85% pure, at least about 85-90% pure, at least about 90-95% pure, or 98-99% pure or purer, e.g., free of contaminants such as cellular debris, macromolecules other than plasminogen, and the like.

### Medicament Formulation

A therapeutic formulation may be prepared by mixing plasminogen of desired purity with an optional pharmaceutical carrier, excipient, or stabilizer (Remington's Pharmaceutical Sciences, 16th edition, Osol, A. ed. (1980)), to form a lyophilized formulation or an aqueous solution. An acceptable carrier, excipient, or stabilizer is non-toxic to a recipient at the employed dosage and concentration, including buffers such as phosphate, citrate and other organic acids; antioxidants such as ascorbic acid and methionine; preservatives (such as octadecyldimethylbenzylammonium chloride; hexanediamine chloride; benzalkonium chloride, benzethonium chloride; phenol, butanol or benzyl alcohol; alkyl parahydroxybenzoate such as methyl or propyl paraben; catechol; resorcinol; cyclohexanol; 3-pentanol; m-cresol); low molecular weight polypeptides (less than about 10 residues); proteins such as serum albumin, gelatin or immunoglobulins; hydrophilic polymers such as polyvinylpyrrolidone; amino acids such as glycine, glutamine, asparagine, histidine, arginine or lysine; monosaccharides, disaccharides and other carbohydrates such as glucose, mannose, or dextrin; chelating agents such as EDTA; carbohydrates such as sucrose, mannitol, fucose, or sorbitol; salt-forming counterions such as sodium; metal complexes (such as zinc-protein complexes); and/or nonionic surfactants such as TWENTM, PLURONICSTM or polyethylene glycol (PEG).

The formulations according to the present application may also contain more than one active compound as required for the particular condition to be treated, preferably those compounds are complementary in activity and do not have side effects with each other. For example, antihypertensive drug, antiarrhythmic drug, drug for treating diabetes, etc.

The plasminogen according to the present application may be encapsulated in microcapsules prepared by techniques such as coacervation or interfacial polymerization, for example, the plasminogen may be placed in colloidal drug delivery systems (e.g., liposomes, albumin microspheres, microemulsions, nanoparticles and nanocapsules) or in hydroxymethyl cellulose or gel-microcapsules and poly-(methyl methacrylate) microcapsules in macroemulsions. These techniques are disclosed in Remington's Pharmaceutical Sciences 16th edition, Osol, A. Ed. (1980).

The plasminogen according to the present application for in vivo administration must be sterile. This may be easily achieved by filtration through sterilizing filters before or after lyophilization and reformulation.

The plasminogen according to the present application may be prepared as a sustained-release formulation. Suitable examples of sustained-release formulations include semipermeable matrices of solid hydrophobic polymers which have a certain shape and contain glycoprotein, for example, membranes or microcapsules. Examples of sustained-release matrices include polyesters, hydrogels such as poly(2-hydroxyethyl-methacrylate) (Langer et al., J. Biomed. Mater. Res., 15:167-277 (1981); Langer, Chem. Tech., 12:98-105 (1982)), or poly(vinyl alcohol), polylactide (US Pat. No.3,773,919, EP58,481), copolymers of L-glutamic acid and γ-ethyl-L-glutamic acid (Sidman, et al., Biopolymers 22:547 (1983)), non-degradable ethylene-vinyl acetate (Langer, et al., supra), or degradable lactic acid-glycolic acid copolymers such as Lupron Depot^{™} (injectable microspheres consisting of lactic acid-glycolic acid copolymer and leuprolide acetate), and poly-D-(-)-3-hydroxybutyric acid. Polymers such as ethylene-vinyl acetate and lactic acid-glycolic acid may release molecules continuously for more than 100 days, while some hydrogels release proteins for shorter period of time. Rational strategies to stabilize proteins may be devised based on the relevant mechanisms. For example, if the mechanism of condensation is found to form intermolecular S-S bond through thiodisulfide interchange, then stabilization may be achieved by modifying sulfhydryl residues, lyophilizing from acidic solutions, controlling humidity, using suitable additives, and developing specific polymer matrix composition.

### Administration and Dosage

Administration of the pharmaceutical composition according to the present application may be accomplished by different means, e.g., nasal inhalation, aerosol inhalation, nasal or eye drop, intravenous administration, intraperitoneal administration, subcutaneous administration, intracranial administration, intrathecal administration, intraarteral administration (e.g., via the carotid artery), intra-rectal administration, intramuscular administration, and rectal administration.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include fluid and nutritional supplements, electrolyte supplements, and the like. Preservatives and other additives may also be present, such as, for example, antimicrobials, antioxidants, chelating agents, and inert gases, etc.

Dosing regimens will be determined by medical personnel based on various clinical factors. As is well known in the medical field, the dosage for any patient depends on a variety of factors, including the patient's size, body surface area, age, the particular compound to be administrated, sex, number and route of administration, general health, and other concomitantly administrated medicaments. The dosage range of the pharmaceutical composition comprising the plasminogen according to the present application may be, for example, about 0.0001-2000 mg/kg, or about 0.001-500 mg/kg (e.g., 0.02 mg/kg, 0.25 mg/kg, 0.5 mg/kg, 0.75 mg/kg, 10 mg/kg, 50 mg/kg, etc.) body weight of the subject per day. For example, the dose may be 1 mg/kg body weight, or 50 mg/kg body weight, or in the range of 1-50 mg/kg, or at least 1 mg/kg. Dosages above or below this exemplary range are also contemplated, especially in view of the factors set forth above. Intermediate doses within the above ranges are also included within the scope of the present application. Subjects may be administrated such doses daily, every other day, weekly, or according to any other schedule determined by empirical analysis. An exemplary dosage schedule includes 0.01-100 mg/kg on consecutive days. Real-time evaluation of therapeutic efficacy and safety is required during the administration of the medicament according to the present application.

### Product or Kit

One embodiment of the present application relates to a product or kit comprising a plasminogen or plasmin according to the application for treating hypertension and a related disease. The product preferably comprises a container, a label or package insert. Suitable containers are bottles, vials, syringes, etc. The container may be made of various materials such as glass or plastic. The container contains a composition which is effective for treatment of the disease or condition according to the present application and has a sterile access port (e.g., the container may be an intravenous solution pack or vial containing a stopper penetrable by a hypodermic needle). At least one active agent in the composition is a plasminogen/ plasmin. The label on or attached to the container indicates that the composition is used for treatment of the hypertension and a related disease mentioned in the present application. The product may further comprise a second container containing a pharmaceutically acceptable buffer, such as phosphate buffered saline, Ringer's solution, and dextrose solution. It may further contain other materials required from a commercial and user standpoint, including other buffers, diluents, filters, needles and syringes. In addition, the product comprises a package insert with instructions for use, including, for example, instructing the user of the composition to administrate the composition comprising a plasminogen to the patient along with other medicaments for treatment of concomitant diseases.

### EXAMPLES

The plasminogen used in the following examples is human plasminogen and is derived from plasma of a human donor, based on methods described in: Kenneth C Robbins, Louis Summaria, David Elwyn et al. Further Studies on the Purification and Characterization of Human Plasminogen and Plasmin. Journal of Biological Chemistry, 1965, 240(1): 541-550; Summaria L, Spitz F, Arzadon L et al. Isolation and characterization of the affinity chromatography forms of human Glu-and Lys-plasminogens and plasmins. J Biol Chem. 1976 Jun 25;251(12):3693-9; HAGAN JJ, ABLONDI FB, DE RENZO EC. Purification and biochemical properties of human plasminogen. J Biol Chem. 1960 Apr; 235:1005-10, with process optimization, being purified from human plasma, with >98% human Lys-plasminogen and Glu-plasminogen.

In the following examples 1-5, db/db mice (purchased from Nanjing Institute of Biomedicine, strain name BKS.Cg-Dock7m+/-Leprdb/JNju) were used as hypertension model to study the effect of plasminogen on hypertension. Several literatures reported that db/db mice spontaneously developed hypertension in 11-14 weeks, and with the increase of age, the blood pressure continued to rise ^{[24,25]}.

### Example 1: Plasminogen Reduces Hypertension in 15-16 Weeks old Diabetic Mice

Twelve 15-16 weeks old db/db male mice were selected. One day before administration of plasminogen, the basal blood pressure was measured after weighing, and they were randomly divided into two groups according to blood pressure, i.e., the vehicle PBS control group and the plasminogen group, with 6 mice in each group. The mice in the plasminogen group were given 2 mg/0.2 ml/mouse/day of plasminogen by tail vein injection, and the mice in the vehicle PBS control group were given the same volume of PBS solution by tail vein injection, for 21 consecutive days. The start of administration was set as day 1, and blood pressure was measured on day 0, 8, 15, and 22; each mouse was continuously measured for five times, and the systolic blood pressure and mean blood pressure of each measurement were recorded. Systolic and mean blood pressure were respectively the mean values of the systolic and mean blood pressure data obtained from the five measurements. Mean blood pressure was calculated as 1/3 systolic pressure + 2/3 diastolic pressure. The blood pressures of the mice were detected by using a non-invasive blood pressure meter (MRBP-M01, IITC Life science).

The results show that, after 21 days of administration, the blood pressure of the mice in the vehicle PBS control group does not change significantly as compared with that before administration, while the systolic blood pressure (Fig. 1A) and the mean blood pressure (Fig. 1B) of the mice in the plasminogen group are significantly decreased; both are significantly lower than those in the vehicle PBS control group, and the statistical difference is significant (* means P<0.05, ** means P<0.01), indicating that plasminogen can significantly reduce hypertension in 15-16 weeks old diabetic mice.

### Example 2: Plasminogen Reduces Hypertension in 25-26 Weeks old Diabetic Mice

Thirteen 25-26 weeks old db/db male mice were selected. One day before administration of plasminogen, the basal blood pressure was measured after weighing, and they were randomly divided into two groups according to blood pressure, i.e., 6 mice in the vehicle PBS control group, and 7 mice in the plasminogen group. The mice in the plasminogen group were given 2mg/0.2 ml/mouse/day of plasminogen by tail vein injection, and the mice in the vehicle PBS control group were given the same volume of PBS solution by tail vein injection, for 21 consecutive days. The start of administration was set as day 1, and blood pressure was measured on day 0, 8, 15, and 22; each mouse was continuously measured for five times, and the systolic blood pressure and mean blood pressure of each measurement were recorded. Systolic and mean blood pressure were respectively the mean values of the systolic and mean blood pressure data obtained from the five measurements. The blood pressures of the mice were detected by using a non-invasive blood pressure meter (MRBP-M01, IITC Life science).

The results show that, after 21 days of administration, the systolic blood pressure of the mice in the vehicle PBS control group does not change significantly as compared with that before administration, while the systolic blood pressure of the mice in the plasminogen group have begun to decrease significantly after 7 days of administration, and is significantly lower than that of the vehicle PBS control group, and the statistical difference is significant (P=0.019); on day 14 and day 21 of the administration, the systolic blood pressure of the two groups of mice is also significantly different (Fig. 2A). After 21 days of administration, the mean blood pressure of mice in the plasminogen group is lower than that in the vehicle PBS control group, and the difference is close to significant (P=0.09) (Fig. 2B), indicating that plasminogen can significantly reduce the high systolic blood pressure in 25-26 weeks old diabetic mice.

### Example 3: Plasminogen Reduces the Level of Serum Angiotensin II in the Model Mice of Diabetic Hypertension

Twelve 15-16 weeks old db/db male mice were selected. One day before administration of plasminogen, the basal blood pressure was measured after weighing, and they were randomly divided into two groups according to blood pressure, i.e., the vehicle PBS control group and the plasminogen group, with 6 mice in each group. The mice in the plasminogen group were given 2 mg/0.2 ml/mouse/day of plasminogen by tail vein injection, and the mice in the vehicle PBS control group were given the same volume of PBS solution by tail vein injection, for 28 consecutive days. On day 29, the eyeballs were removed to collect blood, centrifuging to obtain the supernatant. Serum angiotensin II levels were detected according to the instructions of the angiotensin II detection kit (manufacturer: Wuhan Cusabio Co., Ltd., catalog No.: CSB-E04495m).

The results show that after 28 days of plasminogen administration, the serum angiotensin II level in the plasminogen group is significantly lower than that in the vehicle PBS control group (Fig. 3), indicating that plasminogen can reduce the level of serum angiotensin II in the model mice of diabetic hypertension, thereby correcting hypertension.

### Example 4: Plasminogen Promotes Repair of the Heart Damage in the Model Mice of Diabetic Hypertension

Thirteen 25-26 weeks old db/db male mice were selected. One day before administration of plasminogen, the basal blood pressure was measured after weighing, and they were randomly divided into two groups according to blood pressure, i.e., 6 mice in the vehicle PBS control group, and 7 mice in the plasminogen group. The mice in the plasminogen group were given 2 mg/0.2 ml/mouse/day of plasminogen by tail vein injection, and the mice in the vehicle PBS control group were given the same volume of PBS solution by tail vein injection, for 28 consecutive days. On day 29, the mice were sacrificed to collect the heart to fix in 4% paraformaldehyde for 24 hours. The fixed tissue samples were dehydrated in an ethanol gradient and cleared with xylene before paraffin-embedding. The thickness of the tissue section was 4 µm. The sections were dewaxed and rehydrated, then stained with hematoxylin and eosin (H&E staining). After differentiation with 1% hydrochloric acid in ethanol, the sections were returned to blue in ammonia solution, and then dehydrated with ethanol gradient and sealed. The sections were observed under a 200x optical microscope.

The results show that, compared with the vehicle PBS control group (Fig. 4A), the cardiomyocytes of the mice in the plasminogen group (Fig. 4B) are more compact and arranged more regularly, indicating that plasminogen can significantly alleviate the cardiac injury in the model mice of diabetic hypertension.

### Example 5: Plasminogen Alleviates Renal Fibrosis in the Model Mice of Diabetic Hypertension

Thirteen 25-26 weeks old db/db male mice were selected. One day before administration of plasminogen, the basal blood pressure was measured after weighing, and they were randomly divided into two groups according to blood pressure, i.e., 6 mice in the vehicle PBS control group, and 7 mice in the plasminogen group. The mice in the plasminogen group were given 2 mg/0.2 ml/mouse/day of plasminogen by tail vein injection, and the mice in the vehicle PBS control group were given the same volume of PBS solution by tail vein injection, for 28 consecutive days. On day 29, the mice were sacrificed to collect the kidneys to fix in 4% paraformaldehyde for 24 hours. The fixed tissue samples were dehydrated in an ethanol gradient and cleared with xylene before paraffin-embedding. The thickness of the tissue section was 3 µm. After the sections were dewaxed to water, washing once with water, staining with 0.1% Sirius red saturated picric acid for 30 min, rinsing with running water for 2 min; staining with hematoxylin for 1 min, rinsing with running water, differentiating with 1% hydrochloric acid in ethanol, and returning to blue in ammonia solution, rinsing with running water; then drying and sealing with neutral gum, and finally observing under a 200x optical microscope.

Sirius red staining can permanently stain collagen. As a special staining method for pathological sections, Sirius red staining can specifically display collagen tissue.

The results show that after 28 days of plasminogen administration, the deposition of collagen fibers in the kidneys of the plasminogen group (Fig. 5B) is significantly less than that of the vehicle PBS control group (Fig. 5A), indicating that plasminogen can significantly reduce renal fibrosis in the model mice of diabetic hypertension.

### Example 6: Plasminogen Increases Serum SOD Level in the Model Mice of Angiotensin II-induced Hypertension

Fourteen 21-22 weeks old Plg+/+ male mice were selected to measure blood pressure and body weight. According to blood pressure and body weight, the mice were randomly divided into 3 groups, i.e., 4 mice in blank control group, and 5 mice in each of the PBS control group and plasminogen group. The mice in the blank control group were subcutaneously injected with 0.1 ml of normal saline, and the mice in the vehicle PBS control group and the plasminogen group were subcutaneously injected with 1 mg/kg/mouse/day of angiotensin II, modeling by injection for 14 consecutive days ^{[26]}. The administration of plasminogen or vehicle was started at the same time as the start of modeling, and the mice in the plasminogen group were given 1 mg/0.1 ml/mouse/day of plasminogen by tail vein injection, and the mice in the vehicle PBS control group were given the same volume of PBS solution by tail vein injection, for 14 consecutive days; the mice in the blank control group were not treated. The start of modeling by administration was set as day 1, on day 15 the eyeballs were removed to collect the blood, centrifuging to obtain the supernatant for detecting the level of superoxide dismutase (SOD) in serum. SOD was detected by using SOD detection kit (Nanjing Jiancheng Bioengineering Institute, catalog No.: A001-1), and the detection was carried out according to the method in the instruction manual.

SOD is an important enzyme system for scavenging free radicals in the body. It can scavenge superoxide anion (O²⁻), while O²⁻ is the starting free radical of oxygen free radical. Studies have shown that SOD has a protective effect on hypertension, and makes the level of SOD in patients with hypertension decreased ^{[27]}.

The results show that, there is a certain level of serum SOD in the blank control group, the serum SOD level of the vehicle PBS group is significantly reduced, and the serum SOD level of the plasminogen group is significantly higher than that of the vehicle PBS control group, and the statistical difference is significant (* means P<0.05) (Fig. 6), indicating that plasminogen can enhance the body's ability to scavenge free radicals.

### Example 7: Plasminogen Alleviates Renal Fibrosis in the Model Mice of Angiotensin II-induced Hypertension

Fourteen 21-22 weeks old Plg+/+ male mice were selected to measure blood pressure and body weight. According to blood pressure and body weight, the mice were randomly divided into 3 groups, i.e., 4 mice in blank control group, and 5 mice in each of the PBS control group and plasminogen group. The mice in the blank control group were subcutaneously injected with 0.1 ml of normal saline, and the mice in the vehicle PBS control group and the plasminogen group were subcutaneously injected with 1 mg/kg/mouse/day of angiotensin II, modeling by injection for 14 consecutive days ^{[26]}. The administration of plasminogen or vehicle was started at the same time as the start of modeling, and the mice in the plasminogen group were given 1 mg/0.1 ml/mouse/day of plasminogen by tail vein injection, and the mice in the vehicle PBS control group were given the same volume of PBS solution by tail vein injection, for 14 consecutive days; the mice in the blank control group were not treated. The start of modeling by administration was set as day 1, on day 15 the mice were sacrificed to collect the kidneys to fix in 4% paraformaldehyde for 24 hours. The fixed kidney tissue samples were dehydrated in an ethanol gradient and cleared with xylene before paraffin-embedding. The thickness of the tissue section was 3 µm. After the sections were dewaxed to water, washing once with water, staining with 0.1% Sirius red saturated picric acid for 30 min, rinsing with running water for 2 min; staining with hematoxylin for 1 min, rinsing with running water, differentiating with 1% hydrochloric acid in ethanol, and returning to blue in ammonia solution, rinsing with running water; then drying and sealing with neutral gum, and finally observing under a 200x optical microscope.

The results show that, there is no obvious deposition of collagen fibers in the kidneys of the blank control group (Fig. 7A), and the deposition of collagen fibers (marked by arrows) in the plasminogen group (Fig. 7C) is significantly less than that in the vehicle PBS control group (Fig. 7B), indicating that plasminogen can significantly reduce the renal fibrosis in angiotensin II-induced hypertension model mice, thereby alleviating the renal lesions caused by hypertension.

### Example 8: Plasminogen Alleviates Cardiac Fibrosis in the Model Mice of Angiotensin II-induced Hypertension

Fourteen 21-22 weeks old Plg+/+ male mice were selected to measure blood pressure and body weight. According to blood pressure and body weight, the mice were randomly divided into 3 groups, i.e., 4 mice in blank control group, and 5 mice in each of the PBS control group and plasminogen group. The mice in the blank control group were subcutaneously injected with 0.1 ml of normal saline, and the mice in the vehicle PBS control group and the plasminogen group were subcutaneously injected with 1 mg/kg/mouse/day of angiotensin II, modeling by injection for 14 consecutive days ^{[26]}. The administration of plasminogen or vehicle was started at the same time as the start of modeling, and the mice in the plasminogen group were given 1 mg/0.1 ml/mouse/day of plasminogen by tail vein injection, and the mice in the vehicle PBS control group were given the same volume of PBS solution by tail vein injection, for 14 consecutive days; the mice in the blank control group were not treated. The start of modeling by administration was set as day 1, on day 15 the mice were sacrificed to collect the heart to fix in 4% paraformaldehyde for 24 hours. The fixed cardiac tissue samples were dehydrated in an ethanol gradient and cleared with xylene before paraffin-embedding. The thickness of the tissue section was 3 µm. After the sections were dewaxed to water, washing once with water, staining with 0.1% Sirius red saturated picric acid for 30 min, rinsing with running water for 2 min; staining with hematoxylin for 1 min, rinsing with running water, differentiating with 1% hydrochloric acid in ethanol, and returning to blue in ammonia solution, rinsing with running water; then drying and sealing with neutral gum, and finally observing under a 200x optical microscope.

The results show that there is no obvious deposition of collagen fibers in the heart of the mice in the blank control group (Fig. 8A), and the deposition of collagen fibers (marked by arrows) in the plasminogen group (Fig. 8C) is significantly less than that in the vehicle PBS control group (Fig. 8B), indicating that plasminogen can significantly reduce the cardiac fibrosis in angiotensin II-induced hypertension model mice, thereby alleviating the cardiac disease caused by hypertension.

### Example 9: Plasminogen Alleviates Pulmonary Fibrosis in the Model Mice of Monocrotaline-induced Pulmonary Hypertension

Twelve 12-week-old C57 male mice were weighed, and their blood pressures were measured. According to their blood pressures, they were randomly divided into 2 groups; 4 mice in the blank control group, and 8 mice in the model group. The mice in the blank control group were injected with 100 µl of normal saline through the tail vein, and the mice in the model group were injected with 60 mg/kg/mouse of monocrotaline at a single injection through the tail vein, injecting for 3 consecutive days, and normally feeding the mice ^{[28, 29]}. The blood pressure was measured 3 days later, and the mice in the model group were randomly divided into two groups according to the blood pressure; with 4 mice in each of the vehicle PBS control group and the plasminogen group. The mice in the plasminogen group were given plasminogen by tail vein injection at 1 mg/0.1 ml/mouse/day, and the mice in the vehicle PBS control group were given the same volume of PBS solution by tail vein injection for 28 consecutive days; the mice in the blank control group were not treated with plasminogen. The start of modeling and administration of plasminogen was set as day 1, and on day 29 the mice were sacrificed to collect the lungs and fix in 4% paraformaldehyde fix solution for 24 hours. The fixed lung tissues were dehydrated with ethanol gradient and cleared with xylene before being embedded in paraffin. The thickness of the tissue section was 3 µm. After the sections were dewaxed to water, washing once with water, staining with 0.1% Sirius red saturated picric acid for 30 min, rinsing with running water for 2 min; staining with hematoxylin for 1 min, rinsing with running water, differentiating with 1% hydrochloric acid in ethanol, and returning to blue in ammonia solution, rinsing with running water; then drying and sealing with neutral gum, and finally observing under a 200x optical microscope.

Monocrotaline is a Bispyrrole alkaloid, which is converted by P450 mono-oxygenase in liver and reaches the lungs through blood circulation, causing irreversible damage to the pulmonary blood vessels. Pulmonary vascular endothelial cells were considered to be the target cells of monocrotaline, and endothelial cell injury plays a key role in the process of pulmonary vascular remodeling ^{[28, 29]}.

The results show that, there is basically no collagen deposition in the lungs of the mice in the blank control group (Fig. 9A), and the collagen deposition (marked by arrows) in the lung tissues of the mice in the plasminogen group (Fig. 9C) is significantly less than that in the vehicle PBS control group (Fig. 9B), indicating that plasminogen can significantly reduce the pulmonary fibrosis in the model mice of monocrotaline-induced pulmonary hypertension.

### Example 10: Plasminogen Alleviates Cardiac Fibrosis in the Model Mice of Monocrotaline-induced Pulmonary Hypertension

Twelve 12-week-old C57 male mice were weighed, and their blood pressures were measured. According to their blood pressures, they were randomly divided into 2 groups; 4 mice in the blank control group, and 8 mice in the model group. The mice in the blank control group were injected with 100 µl of normal saline through the tail vein, and the mice in the model group were injected with 60 mg/kg/mouse of monocrotaline at a single injection through the tail vein, injecting for 3 consecutive days, and normally feeding the mice ^{[28, 29]}. The blood pressure was measured 3 days later, and the mice in the model group were randomly divided into two groups according to the blood pressure; with 4 mice in each of the vehicle PBS control group and the plasminogen group. The mice in the plasminogen group were given plasminogen by tail vein injection at 1 mg/0.1 ml/mouse/day, and the mice in the vehicle PBS control group were given the same volume of PBS solution by tail vein injection for 28 consecutive days; the mice in the blank control group were not treated with plasminogen. The start of modeling and administration of plasminogen was set as day 1, and on day 29 the mice were sacrificed to collect the heart and fix in 4% paraformaldehyde fix solution for 24 hours. The fixed cardiac tissues were dehydrated with ethanol gradient and cleared with xylene before being embedded in paraffin. The thickness of the tissue section was 3 µm. After the sections were dewaxed to water, washing once with water, staining with 0.1% Sirius red saturated picric acid for 30 min, rinsing with running water for 2 min; staining with hematoxylin for 1 min, rinsing with running water, differentiating with 1% hydrochloric acid in ethanol, and returning to blue in ammonia solution, rinsing with running water; then drying and sealing with neutral gum, and finally observing under a 200x optical microscope.

The results show that, there is basically no collagen deposition in the hearts of the mice in the blank control group (Fig. 10A), and the collagen deposition (marked by arrows) in the heart of the mice in the plasminogen group (Fig. 10C) is significantly less than that in the vehicle PBS control group (Fig. 10B), indicating that plasminogen can significantly reduce the cardiac fibrosis in the model mice of monocrotaline-induced pulmonary hypertension.

### Example 11: Plasminogen Alleviates Kidney Fibrosis in the Model Mice of Monocrotaline-induced Pulmonary Hypertension

Twelve 12-week-old C57 male mice were weighed, and their blood pressures were measured. According to their blood pressures, they were randomly divided into 2 groups; 4 mice in the blank control group, and 8 mice in the model group. The mice in the blank control group were injected with 100 µl of normal saline through the tail vein, and the mice in the model group were injected with 60 mg/kg/mouse of monocrotaline at a single injection through the tail vein, injecting for 3 consecutive days, and normally feeding the mice ^{[28, 29]}. The blood pressure was measured 3 days later, and the mice in the model group were randomly divided into two groups according to the blood pressure; with 4 mice in each of the vehicle PBS control group and the plasminogen group. The mice in the plasminogen group were given plasminogen by tail vein injection at 1 mg/0.1 ml/mouse/day, and the mice in the vehicle PBS control group were given the same volume of PBS solution by tail vein injection for 28 consecutive days; the mice in the blank control group were not treated with plasminogen. The start of modeling and administration of plasminogen was set as day 1, and on day 29 the mice were sacrificed to collect the kidneys and fix in 4% paraformaldehyde fix solution for 24 hours. The fixed kidney tissues were dehydrated with ethanol gradient and cleared with xylene before being embedded in paraffin. The thickness of the tissue section was 3 µm. After the sections were dewaxed to water, washing once with water, staining with 0.1% Sirius red saturated picric acid for 30 min, rinsing with running water for 2 min; staining with hematoxylin for 1 min, rinsing with running water, differentiating with 1% hydrochloric acid in ethanol, and returning to blue in ammonia solution, rinsing with running water; then drying and sealing with neutral gum, and finally observing under a 200x optical microscope.

The results show that there is basically no collagen deposition in the kidneys of the mice in the blank control group (Fig. 11A), and the collagen deposition (marked by arrows) in the kidneys of the mice in the plasminogen group (Fig. 11C) is significantly less than that in the vehicle PBS control group (Fig. 11B), indicating that plasminogen can significantly reduce the kidney fibrosis in the model mice of monocrotaline-induced pulmonary hypertension.

### Example 12: Plasminogen Alleviates Pulmonary Liver Fibrosis in the Model Mice of Monocrotaline-induced Pulmonary Hypertension

Twelve 12-week-old C57 male mice were weighed, and their blood pressures were measured. According to their blood pressures, they were randomly divided into 2 groups; 4 mice in the blank control group, and 8 mice in the model group. The mice in the blank control group were injected with 100 µl of normal saline through the tail vein, and the mice in the model group were injected with 60 mg/kg/mouse of monocrotaline at a single injection through the tail vein, injecting for 3 consecutive days, and normally feeding the mice ^{[28, 29]}. The blood pressure was measured 3 days later, and the mice in the model group were randomly divided into two groups according to the blood pressure; with 4 mice in each of the vehicle PBS control group and the plasminogen group. The mice in the plasminogen group were given plasminogen by tail vein injection at 1 mg/0.1 ml/mouse/day, and the mice in the vehicle PBS control group were given the same volume of PBS solution by tail vein injection for 28 consecutive days; the mice in the blank control group were not treated with plasminogen. The start of modeling and administration of plasminogen was set as day 1, and on day 29 the mice were sacrificed to collect the livers and fix in 4% paraformaldehyde fix solution for 24 hours. The fixed liver tissues were dehydrated with ethanol gradient and cleared with xylene before being embedded in paraffin. The thickness of the tissue section was 3 µm. After the sections were dewaxed to water, washing once with water, staining with 0.1% Sirius red saturated picric acid for 30 min, rinsing with running water for 2 min; staining with hematoxylin for 1 min, rinsing with running water, differentiating with 1% hydrochloric acid in ethanol, and returning to blue in ammonia solution, rinsing with running water; then drying and sealing with neutral gum, and finally observing under a 200x optical microscope.

The results show that there is basically no collagen deposition in the livers of the mice in the blank control group (Fig. 12A), and the collagen deposition (marked by arrows) in the livers of the mice in the plasminogen group (Fig. 12C) is significantly less than that in the vehicle PBS control group (Fig. 12B), indicating that plasminogen can significantly reduce the liver fibrosis in the model mice of monocrotaline-induced pulmonary hypertension.

### Example 13: Plasminogen Promotes Elevation of Blood Pressure in the Mice with Ischemic Renal Atrophy

Eighteen 6-7 weeks old C57 male mice were randomly divided into two groups, 4 mice in the blank control group, and 14 mice in the model group. Mice in the blank control group do not receive any treatment. The mice in the model group were anesthetized by intraperitoneal injection of 3% sodium pentobarbital (50 mg/kg), and the skin, fascia, and muscle layers were incised at the midline of the abdomen by surgical methods, and the intestines, pancreas and other organs were moved away to expose the surgical field, the aorta and cardinal vein in the renal pelvis of the left kidney were bluntly separated, and the separated blood vessels were ligated with No. 5 silk thread. After ensuring that the ligation was complete, the intestines, pancreas and other organs were put back to their corresponding physiological positions, continuously suturing the muscle layer by surgical methods, the skin was sutured by the nodal suture (interrupted suture) method. After completing the suture, the wound was disinfected with medical iodophor, and the animal were placed on a heating pad at 37°C to observe and wait for it to regain consciousness. Drinking water should not be given until the animal was fully awake. Antibiotics (5000U/mouse) was given by intramuscular injection, painkiller (2mg/kg) was given by subcutaneous injection, and the injections were performed continuously for 3 days. 14 days after surgical ligation, the mice in the model group were anesthetized by intraperitoneal injection of 3% sodium pentobarbital, the abdomen was opened to loosen the ligation line of the left renal blood vessel, then the abdominal cavity was quickly sutured, and the wound was disinfected with medical iodophor, and the animal were placed on a heating pad at 37°C to observe and wait for it to regain consciousness. Drinking water should not be given until the animal was fully awake. Antibiotics (5000U/mouse) was given by intramuscular injection, painkiller (2mg/kg) was given by subcutaneous injection, and the injections were performed continuously for 3 days ^{[30]}. After the sutures were removed, all the mice were weighed, and the mice in the model group were randomly divided into two groups, 7 mice in each of the plasminogen group and the PBS control group; the administration was started, and the start of administration was set as day 1. The mice in the plasminogen group were given 1 mg/0.1 ml/mouse/day of plasminogen by tail vein injection, and the mice in the vehicle PBS control group were given the same volume of PBS by tail vein injection, performing the administration for 14 consecutive days; on day 15, the blood pressures (systolic blood pressure and mean blood pressure) of the mice were detected by using a non-invasive blood pressure meter (MRBP-M01, IITC Life science).

The results show that, the systolic blood pressure and mean blood pressure of the plasminogen group and the blank control group are significantly higher than the corresponding blood pressures of the PBS control group, and the statistical difference between the plasminogen group and the PBS control group is significant (* means P<0.05) (Fig. 13), indicating that plasminogen can promote the hypotension caused by renal atrophy to return to normal level.

### Example 14: Plasminogen Reduces Blood Pressure in the Model Mice of Angiotensin II-induced Hypertension

Twenty-one 8-9 weeks old C57 male mice were randomly divided into three groups, i.e., blank control group, vehicle group and plasminogen group, with 7 mice in each group. Mice in the blank control group do not receive any treatment. In the plasminogen group and vehicle group, all mice were injected subcutaneously with angiotensin II solution (0.25 mg/ml) at a dose of 1 mg/kg/mouse, twice a day, with an interval of 8 hours, modeling for 6 continuous days ^{[31]}. 2 hours after the first injection of angiotensin II for modeling, the administration was started in the plasminogen group and the vehicle group and recorded as day 1, i.e., the mice in the plasminogen group were administrated with 1 mg/0.1 ml/mouse/day of plasminogen by tail vein injection, the same volume of vehicle was injected into the mice in the vehicle group by tail vein injection, and the administration was performed for 5 continuous days; while the mice in the blank control group were not treated. On day 6, blood pressure of all mice was measured. Five consecutive measurements were made for each mouse, and the systolic and mean blood pressures were recorded for each measurement. Systolic and mean blood pressure were respectively the mean values of systolic and mean blood pressure data obtained from five measurements. The blood pressures of the mice were detected by using a non-invasive blood pressure meter (MRBP-M01, IITC Life science).

The results show that, the mean blood pressure and systolic blood pressure of the mice in the vehicle group are significantly increased, and the mean blood pressure and systolic blood pressure of the mice in the plasminogen group are significantly lower than those of the mice in the vehicle group, and the statistical difference is significant (* means P<0.05, ** means P <0.01) (Fig. 14), indicating that plasminogen can reduce blood pressure in the model mice of hypertension.

### Example 15: Plasminogen Promotes Elevation of the Level of Angiotensin-converting Enzyme 2 in the Model Mice of Diabetic Hypertension

Twelve 24-25 weeks old db/db male mice were selected. One day before administration, the basal blood pressure was measured after weighing, and the mice were randomly divided into two groups according to blood pressure, i.e., 6 mice in each of the vehicle group and the plasminogen group. Mice in the plasminogen group were given plasminogen by tail vein injection at 2 mg/0.2 ml/mouse/day, and mice in the vehicle group were given the same volume of PBS solution by tail vein injection, the administration was performed for 28 consecutive days. On day 29, the eyeballs were removed to collect blood, centrifuging to obtain the supernatant. Serum ACE2 levels were detected according to the instructions of the angiotensin-converting enzyme 2 (ACE2) detection kit (manufacturer: Wuhan Cusabio Co., Ltd., catalog No.: CSB-E17204m).

Angiotensin-converting enzyme 2 (ACE2) is an important negative regulator of the renin-angiotensin system, and is the main pathway for degradation of angiotensin II (AngII) in vivo. Under the action of ACE2, AngII is converted into AngI-7; in addition, ACE2 can also catalyze the degradation of AngI into Ang(1-9), which is converted into Angl-7 by ACE. The affinity of ACE2 for AngII is 400 times that for Ang I, and the main function of ACE2 is to catalyze the conversion of AngII to Ang(1-7). The physiological role of ACE2 is related to hypertension, cardiac function and diabetes, and acts as a receptor for severe acute respiratory syndrome coronavirus ^{[12]}.

The results show that, the serum ACE2 level of the mice in the plasminogen group is significantly higher than that in the vehicle group, and the statistical difference is close to significant (P=0.057) (Fig. 15), indicating that plasminogen can promote the increase of serum ACE2 level in the model mice of diabetic hypertension.

### Example 16: Plasminogen Reduces Serum ACE2 Level in the Model Mice of Angiotensin II-induced Hypertension

Twenty-one 8-9 weeks old C57 male mice were randomly divided into three groups, i.e., 7 mice in each of the blank control group, the vehicle group, and the plasminogen group. After grouping the mice in the plasminogen group and the vehicle group, they were subcutaneously injected with angiotensin II solution (0.25 mg/ml) at a dose of 1 mg/kg/mouse, twice a day, with an interval of 8 hours, modeling for 7 consecutive days. The preparation of Angiotensin II solution: firstly, angiotensin II powder (catalog No.: A9292-10mg, manufacturer: Beijing Solarbio Biotech Co., Ltd.) was taken to dissolve in 1ml of deionized water, preparing a 10mg/ml solution, and dividing it to avoid repeated freezing and thawing; the solution was diluted 40 times when using it, preparing a 0.25mg/ml ready-to-use solution; the stock solution was stored at -20°C. 2 hours after the first injection for modeling, administration was started in the plasminogen group and the vehicle group, and recorded as day 1. The mice in the plasminogen group were injected with plasminogen by tail vein at 1 mg/0.1ml/mouse/day, and the mice in the vehicle group were injected with the same volume of the vehicle by tail vein, the administration was performed for 7 consecutive days, and the blank control group was not treated. On day 8, the eyeballs were removed to collect blood, centrifuging to obtain the supernatant. Serum ACE2 levels were detected according to the instructions of angiotensin-converting enzyme 2 (ACE2) detection kit (manufacturer: Shanghai Ximei Chemical Co., Ltd., catalog No.: CSB-E17204m).

The results show that, there is a certain level of ACE2 in the blood of the mice in the blank control group, the level of ACE2 in the blood of the mice in the vehicle group is significantly higher than that of the mice in the blank control group, and the level of ACE2 in the blood of the mice in the plasminogen group is significantly lower than that of the mice in the vehicle group; and the statistical difference is extremely significant (** means P<0.01) (Fig. 16), indicating that plasminogen can promote the decrease of serum ACE2 level in angiotensin II-induced hypertension model mice.

### Example 17: Plasminogen Reduces Serum ACE Level in the Model Mice of Angiotensin II-induced Hypertension

Twenty-one 8-9 weeks old C57 male mice were randomly divided into three groups, i.e., 7 mice in each of the blank control group, the vehicle group, and the plasminogen group. After grouping the mice in the plasminogen group and the vehicle group, they were subcutaneously injected with angiotensin II solution (0.25 mg/ml) at 1 mg/kg/mouse, twice a day, with an interval of 8 hours, modeling for 7 consecutive days. The preparation of Angiotensin II solution: firstly, angiotensin II powder (catalog No.: A9292-10mg, manufacturer: Beijing Solarbio Biotech Co., Ltd.) was taken to dissolve in 1ml of deionized water, preparing a 10mg/ml solution, and dividing it to avoid repeated freezing and thawing; the solution was diluted 40 times when using it, preparing a 0.25mg/ml ready-to-use solution; stock solution was stored at -20°C. 2 hours after the first injection for modeling, administration was started in the plasminogen group and the vehicle group, and recorded as day 1. The mice in the plasminogen group were injected with plasminogen by tail vein at 1 mg/0.1ml/mouse/day, and the mice in the vehicle group were injected with the same volume of the vehicle by tail vein, the administration was performed for 7 consecutive days, and the blank control group was not treated. On day 8, the eyeballs were removed to collect blood, centrifuging to obtain the supernatant. Serum ACE levels were detected according to the instructions of angiotensin-converting enzyme (ACE) detection kit (manufacturer: Wuhan Cusabio Co., Ltd., catalog No.: CSB-E04492m).

Angiotensin-converting enzyme (ACE) is a key enzyme in the renin-angiotensin system and plays a key role in the production of AngII ^{[32]}.

The results show that, there is a certain level of ACE in the blood of the mice in the blank control group, the level of ACE in the blood of the mice in the vehicle group is significantly higher than that of the mice in the blank control group, and the level of ACE in the blood of the mice in the plasminogen group is significantly lower than that of the mice in the vehicle group; and the statistical difference is close to significant (P=0.051) (Fig. 17), indicating that plasminogen can promote the decrease of serum ACE level in the model mice AngII-induced hypertension.

### Example 18: Therapeutic Effects of Plasminogen on Clinical Volunteers

The following is the data of clinical volunteers, all patients had signed the informed consent form to voluntarily take medication, and obtained the approval of the hospital ethics committee. The plasminogen used below was diluted with physiological saline at a concentration of 5 or 10 mg/ml. In the case of administrating plasminogen by aerosol inhalation, the plasminogen solution should be nebulized with a nebulizer before use.

### Case 1

The patient, female, 72 years old, had a history of hypertension for more than 30 years. The systolic blood pressure was 160mmHg and occasionally 180mmHg. After taking antihypertensive drugs, the blood pressure was controlled at about 130/80mmHg. The patient received intravenous injection of human plasminogen once a day, starting at a dose of 50 mg/time and gradually increasing to 135 mg/time. The patient's systolic blood pressure decreased to 141 mmHg from the day 6 after administration, and the blood pressure fluctuated unstable (normal and abnormal alternation) during the period from day 6 to day 11; after day 12, the blood pressure decreased and remained normal and stable for a week. The patient did not take any antihypertensive drugs while receiving the above intravenous plasminogen therapy. The blood pressure of the patient during the treatment period is shown in Fig. 18, indicating that plasminogen can reduce the blood pressure of hypertensive patients, making it close to the normal level.

### Case 2

The patient, female, 79 years old, had coronary heart disease for more than 20 years, and had cerebral infarction in 2014, a diabetes history of 23 years, long-term insulin injection. She had been diagnosed with hypertension for 5 years, after taking antihypertensive drugs, the blood pressure was 130-150/50-60mmHg in the morning, and about 150/60mmHg in the afternoon. The patient was administrated with human plasminogen by intravenous injection, once a day, at a dose of 100 mg/time.

The patient's blood pressure was 135/54 mmHg on day 13 after administration, and the blood pressure was also stabilized at the normal level in the later period without antihypertensive drugs. The blood pressure of the patient during medication is shown in Fig. 19.

### Case 3

The patient, female, 76 years old, had a history of diabetes for 20 years and a history of hypertension for 10 years. The highest blood pressure was 168/70mmHg, with taking nifedipine sustained-release tablets orally, the blood pressure was controlled at about 114/55mmHg, and the diastolic blood pressure was lower than the normal value. Human plasminogen was administered to the patient by intravenous injection, 1-2 times a day, with a daily dosage of 35-75 mg.

After the administration, the diastolic blood pressure reached a normal value from less than 60mmHg, and the blood pressure was normal and stable during the treatment; after the treatment, the antihypertensive drug was halved, and the blood pressure was basically maintained at 130-140/64-76mmHg. The blood pressure of the patient during the treatment period is shown in Fig. 20.

Plasminogen can promote the normalization of blood pressure in a hypertensive patient, and can reduce the dosage of antihypertensive drug.

### Case 4

The patient, male, 57 years old, was admitted to the hospital for re-examination, and found that the abdominal aorta was thickened and diagnosed as aortic dissection. The hospital recommended surgery. The patient had a family history of heart disease, and hypertension for more than 10 years. The patient was allergic to contrast media, and had eczema and hives. Blood pressure was high in the morning and evening, the highest systolic blood pressure might reach 200mmHg, and the pressure difference was large, which might reach more than 80mmHg, with poor sleep, falling asleep slowly, getting up 3-4 times at night, affected sleep.

Human plasminogen was administered by intravenous injection, 1-2 times a day, at a dosage of 50-150 mg/day.

On day 5 after administration, blood pressure began to drop in the morning and evening, and the pressure difference decreased. The patient reported that the blood pressure did not drop below 140/75mmHg while taking antihypertensive drug before the treatment, and this was the first time it had improved. The blood pressure was maintained at about 136/73mmHg in the morning and evening on day 6 after administration.

On day 7 after administration, the blood pressure remained stable in the morning and evening, and was around 130/70 mmHg. The blood pressure of the patient during the treatment period is shown in Fig. 21.

In addition, on day 5 after administration, the patient's sleep was significantly improved, and he did not wake up at night. Eczema and urticaria symptoms were also improved significantly. Since then, the patient's symptoms have continued to improve.

### Case 5

The patient, 78 years old, was diagnosed as critically ill with novel coronavirus pneumonia. During admission, his blood pressure was found to have risen to 150/78 mmHg, and he had no history of hypertension and diagnostic check. Human plasminogen was administered to the patient by inhalation, 2 times a day, 10 mg each time. Blood pressure returned to normal after 1 day. The blood pressure of the patient during the treatment period is shown in Table 1 and Table 2.

It indicates that human plasminogen can promote the recovery of hypertension in critically ill patients with novel coronavirus pneumonia.

**Table 1: the test results of blood pressure in critically ill patients with new coronavirus pneumonia on the first day of administration**

| Items | Before the first aerosol inhalation | Before the second aerosol inhalation | After aerosol inhalation |
|---|---|---|---|
| Blood pressure (mmHg) | | 150/78 | 150/78 |

**Table 2: the test results of blood pressure in critically ill patients with new coronavirus pneumonia on the second day of administration**

| Items | Before or after the first aerosol inhalation | Before the second aerosol inhalation | After aerosol inhalation |
|---|---|---|---|
| Blood pressure (mmHg) | 129/61 | 130/64 | 130/64 |

### Case 6

The patient was diagnosed as critically ill with new coronavirus pneumonia. In addition to relevant clinical symptoms, his blood pressure was 139/94 mmHg, and his diastolic blood pressure was higher than normal value. Plasminogen was administered to the patient by aerosol inhalation, 2 times a day, 10 mg each time. Diastolic blood pressure returned to normal after 1 treatment. The blood pressure of the patient during the treatment period is shown in Table 3.

Plasminogen can promote the return of hypertension to normal in a critically ill patient with novel coronavirus pneumonia.

**Table 3: the test results of blood pressure in critically ill patients with new coronavirus pneumonia during medication**

| Items | Before the first treatment | Before the second treatment | 1 hour after treatment |
|---|---|---|---|
| Blood pressure (mmHg) | 139/94 | 130/87 | 120/83 |

### Case 7

Patient, female, 67 years old, was bedridden, and unable to speak due to tracheotomy; she had no history of diabetes, heart disease, and hypertension; had no history of allergies; she had Parkinson's disease for 6 years, was unable to walk for 4 years, and bedridden for 3 years with catheterization for 1.5 years. The patient could not turn over on her own, and could simply move her limbs by herself and continuously inhale oxygen at a low flow every day with blood pressure of 80/45mmHg. She was diagnosed with hypotension and Parkinson's. Plasminogen was administered to the patient by intravenous injection, once a day, at a dose of 50-250 mg/day, starting at 50 mg/day and gradually increasing thereafter. The administration of plasminogen was performed for 7 consecutive days, then continuing the administration on day 9, day 11 and day 13; and stopping the administration on day 8, day 10 and day 12 for observation.

### Therapeutic Effects:

Blood pressure showed an upward trend after administration of plasminogen, and normal blood pressure appeared for the first time on day 3 of treatment. Blood pressure was remained normal from day 9 after the administration, and remained at 95/70 mmHg after stopping administration. The detection results of blood pressure of the patient during the treatmnt period are shown in Fig. 22.

It indicates that administration of plasminogen can promote blood pressure recovery in a hypotensive patient.

### Case 8

The patient, female, 50 years old, had hypotension for many years with a low blood pressure of 80/60mmHg in ordinary days, and sometimes had dizziness after getting up in the morning. She was diagnosed with hypotension.

Therapeutic regimen: intravenous injection, once a day with a dosage of 100mg.

### Therapeutic Effects:

The patient's blood pressure reached normal on day 2 after administration of plasminogen, and reached about 110/70mmHg one week later. The detection results of blood pressure of the patient during the medication period are shown in Fig. 23.

It indicates that administration of plasminogen can promote blood pressure recovery in a hypotensive patient.

## Claims

1. A pharmaceutical composition comprising an effective amount of component of plasminogen activation pathway for use in treating an abnormal blood pressure condition in a subject suffering from the abnormal blood pressure condition, wherein the abnormal blood pressure condition is hypertension,
wherein the component of plasminogen activation pathway is selected from the group consisting of: plasminogen, recombinant human plasmin, Lys-plasminogen, Glu-plasminogen, plasmin, a variant or an analog of plasminogen or plasmin comprising one or more kringle domains and protease domains of plasminogen and plasmin, mini-plasminogen, mini-plasmin, micro-plasminogen, micro-plasmin, delta-plasminogen, delta-plasmin, tPA and uPA.

2. The pharmaceutical composition for use according to claim 1, wherein the compound further alleviates tissue and organ damage or a complication thereof caused by hypertension.

3. The pharmaceutical composition for use according to claim 2, wherein the complication caused by abnormal blood pressure is caused by hypertension and is one or more selected from the group consisting of: arrhythmia, heart failure, coronary heart disease, cerebral hemorrhage, cerebral thrombosis, cerebral infarction, hypertensive nephropathy, renal failure, uremia, liver cirrhosis, pulmonary hypertension, pulmonary fibrosis and microthrombosis.

4. The pharmaceutical composition for use according to claims 1 to 3, wherein the composition can reduce tissue organ fibrosis, wherein said reducing tissue organ fibrosis is reducing fibrosis of heart tissue, lung tissue, kidney tissue or liver tissue.

5. The pharmaceutical composition for use according to any one of claims 1-4, wherein the compound is plasminogen,
preferably, the plasminogen has at least 75%, 80%, 85%, 90%, 95%, 96%, 97%, 98% or 99% sequence identity with SEQ ID NO: 2, and still has the proteolytic or lysine-binding activity of plasminogen.

6. The pharmaceutical composition for use according to any one of claims 1-5, wherein the plasminogen comprises the plasminogen active fragment represented by SEQ ID NO: 14, and has the proteolytic activity of plasminogen.

7. The pharmaceutical composition for use according to any one of claims 1-6, wherein the plasminogen is selected from the group consisting of: Glu-plasminogen, Lys-plasminogen, mini-plasminogen, micro-plasminogen, delta-plasminogen, or a variant thereof retaining the proteolytic activity of plasminogen.

8. The pharmaceutical composition for use according to any one of claims 1-7, wherein the plasminogen is natural or synthetic human plasminogen, or a variant or fragment thereof which retains the proteolytic or lysine-binding activity of plasminogen.

9. The pharmaceutical composition for use according to any one of claims 1-8, wherein the compound is used in combination with one or more other therapeutic methods or medicaments.

10. The pharmaceutical composition for use according to claim 8, wherein the other medicament is used for the treatment of a disease other than the abnormal blood pressure condition in the subject with the abnormal blood pressure condition.

11. The pharmaceutical composition for use according to any one of claims 1-10, wherein the compound is administered by nasal inhalation, aerosol inhalation, nasal drop, ear drop or eye drop, and intravenous administration, intraperitoneal administration, subcutaneous administration, intracranial administration, intrathecal administration, intra-arterial administration, intra-rectal administration and/or intramuscular administration.

## Patentansprüche

1. Pharmazeutische Zusammensetzung, umfassend eine wirksame Menge einer Komponente des Plasminogen-Aktivierungswegs zur Verwendung bei der Behandlung eines anormalen Blutdruckzustands bei einem Patienten, der an dem anormalen Blutdruckzustand leidet, wobei der anormale Blutdruckzustand Bluthochdruck ist, wobei die Komponente des Plasminogen-Aktivierungsweges ausgewählt ist aus der Gruppe bestehend aus: Plasminogen, rekombinantem menschlichem Plasmin, Lys-Plasminogen, Glu-Plasminogen, Plasmin, einer Variante oder einem Analogon von Plasminogen oder Plasmin, das eine oder mehrere Kringle-Domänen und Protease-Domänen von Plasminogen und Plasmin umfasst, Mini-Plasminogen, Mini-Plasmin, Mikro-Plasminogen, Mikro-Plasmin, Delta-Plasminogen, Delta-Plasmin, tPA und uPA.

2. Pharmazeutische Zusammensetzung zur Verwendung gemäß Anspruch 1, wobei die Verbindung ferner durch Bluthochdruck verursachte Gewebe- und Organschäden oder eine Komplikation davon lindert.

3. Pharmazeutische Zusammensetzung zur Verwendung gemäß Anspruch 2, wobei die durch abnormalen Blutdruck verursachte Komplikation durch Bluthochdruck verursacht wird und eine oder mehrere ist, ausgewählt aus der Gruppe bestehend aus: Arrhythmie, Herzinsuffizienz, koronare Herzerkrankung, zerebrale Blutung, zerebrale Thrombose, zerebraler Infarkt, hypertensive Nephropathie, Nierenversagen, Urämie, Leberzirrhose, pulmonale Hypertonie, pulmonale Fibrose und Mikrothrombose.

4. Pharmazeutische Zusammensetzung zur Verwendung nach den Ansprüchen 1 bis 3, wobei die Zusammensetzung die Organgewebefibrose verringern kann, wobei die Verringerung der Organgewebefibrose die Verringerung der Fibrose von Herzgewebe, Lungengewebe, Nierengewebe oder Lebergewebe ist.

5. Pharmazeutische Zusammensetzung zur Verwendung nach einem der Ansprüche 1-4, wobei die Verbindung Plasminogen ist, vorzugsweise hat das Plasminogen mindestens 75 %, 80 %, 85 %, 90 %, 95 %, 96 %, 97 %, 98 % oder 99 % Sequenzidentität mit SEQ ID NO: 2 und besitzt noch die proteolytische oder lysinbindende Aktivität von Plasminogen.

6. Pharmazeutische Zusammensetzung zur Verwendung nach einem der Ansprüche 1-5, wobei das Plasminogen das durch SEQ ID NO: 14 dargestellte plasminogenaktive Fragment umfasst und die proteolytische Aktivität von Plasminogen aufweist.

7. Pharmazeutische Zusammensetzung zur Verwendung nach einem der Ansprüche 1-6, wobei das Plasminogen ausgewählt ist aus der Gruppe bestehend aus: Glu-Plasminogen, Lys-Plasminogen, Mini-Plasminogen, Mikro-Plasminogen, Delta-Plasminogen oder einer Variante davon, die die proteolytische Aktivität von Plasminogen beibehält.

8. Pharmazeutische Zusammensetzung zur Verwendung nach einem der Ansprüche 1-7, wobei das Plasminogen natürliches oder synthetisches menschliches Plasminogen ist oder eine Variante oder ein Fragment davon, dass die proteolytische oder lysinbindende Aktivität von Plasminogen beibehält.

9. Pharmazeutische Zusammensetzung zur Verwendung nach einem der Ansprüche 1-8, wobei die Verbindung in Kombination mit einem oder mehreren anderen therapeutischen Verfahren oder Medikamenten verwendet wird.

10. Pharmazeutische Zusammensetzung zur Verwendung nach Anspruch 9, wobei das andere Medikament zur Behandlung einer anderen Krankheit als dem anormalen Blutdruckzustand bei dem Patienten mit dem anormalen Blutdruckzustand verwendet wird.

11. Pharmazeutische Zusammensetzung zur Verwendung nach einem der Ansprüche 1-10, wobei die Verbindung durch nasale Inhalation, Aerosol-Inhalation, Nasentropfen, Ohrentropfen oder Augentropfen, und intravenöse Verabreichung, intraperitoneale Verabreichung, subkutane Verabreichung, intrakranielle Verabreichung, intrathekale Verabreichung, intraarterielle Verabreichung, intrarektale Verabreichung und/oder intramuskuläre Verabreichung verabreicht wird.

## Revendications

1. Composition pharmaceutique comprenant une quantité efficace de composant d'une voie d'activation du plasminogène pour une utilisation dans le traitement d'un problème de pression artérielle anormale chez un sujet souffrant d'un problème de pression artérielle anormale, dans laquelle le problème de pression artérielle anormale est l'hypertension, dans laquelle le composant d'une voie d'activation du plasminogène est choisi dans le groupe constitué par : plasminogène, plasmine humaine recombinante, Lys-plasminogène, Glu-plasminogène, plasmine, un variant ou un analogue du plasminogène ou de la plasmine comprenant un ou plusieurs domaines kringle et domaines de protéase du plasminogène et de la plasmine, miniplasminogène, miniplasmine, microplasminogène, microplasmine, deltaplasminogène, deltaplasmine, tPA et uPA.

2. Composition pharmaceutique pour utilisation selon la revendication 1, dans laquelle le composé atténue en outre les dommages aux tissus et aux organes ou une complication de ceux-ci causés par l'hypertension.

3. Composition pharmaceutique destinée à être utilisée selon la revendication 2, dans laquelle la complication causée par une pression artérielle anormale est causée par l'hypertension et est une ou plusieurs complications choisies dans le groupe constitué par : arythmie, insuffisance cardiaque, maladie coronarienne, hémorragie cérébrale, thrombose cérébrale, infarctus cérébral, néphropathie hypertensive, insuffisance rénale, urémie, cirrhose du foie, hypertension pulmonaire, fibrose pulmonaire et microthrombose.

4. Composition pharmaceutique pour utilisation selon les revendications 1 à 3, dans laquelle la composition peut réduire la fibrose des organes tissulaires, dans laquelle ladite réduction de la fibrose des organes tissulaires est une réduction de la fibrose du tissu cardiaque, du tissu pulmonaire, du tissu rénal ou du tissu hépatique.

5. Composition pharmaceutique pour utilisation selon l'une quelconque des revendications 1 à 4, dans laquelle le composé est le plasminogène, de préférence, le plasminogène a au moins 75 %, 80 %, 85 %, 90 %, 95 %, 96 %, 97 %, 98 % ou 99 % d'identité de séquence avec la SEQ ID NO: 2, et a toujours l'activité protéolytique ou de liaison à la lysine de plasminogène.

6. Composition pharmaceutique pour utilisation selon l'une quelconque des revendications 1 à 5, dans laquelle le plasminogène comprend le fragment actif de plasminogène représenté par la SEQ ID NO: 14, et a l'activité protéolytique de plasminogène.

7. Composition pharmaceutique pour utilisation selon l'une quelconque des revendications 1 à 6, dans laquelle le plasminogène est choisi dans le groupe constitué par : Glu-plasminogène, Lys-plasminogène, miniplasminogène, microplasminogène, deltaplasminogène, ou un variant de ceux-ci conservant l'activité protéolytique de plasminogène.

8. Composition pharmaceutique pour une utilisation selon l'une quelconque des revendications 1 à 7, dans laquelle le plasminogène est du plasminogène humain naturel ou synthétique, ou un variant ou un fragment de celui-ci, qui conserve l'activité protéolytique ou de liaison à la lysine de plasminogène.

9. Composition pharmaceutique pour une utilisation selon l'une quelconque des revendications 1 à 8, dans laquelle le composé est utilisé en combinaison avec un ou plusieurs autres procédés thérapeutiques et/ou médicaments.

10. Composition pharmaceutique pour utilisation selon la revendication 8, dans laquelle l'autre médicament est utilisé pour le traitement d'une maladie autre que le problème de pression artérielle anormale chez le sujet présentant le problème de pression artérielle anormale.

11. Composition pharmaceutique pour une utilisation selon l'une quelconque des revendications 1 à 10, dans laquelle le composé est administré par inhalation nasale, inhalation d'aérosol, goutte nasale, goutte auriculaire ou goutte oculaire, et administration intraveineuse, administration intrapéritonéale, administration sous-cutanée, administration intracrânienne, administration intrathécale, administration intra-artérielle, administration intrarectale et/ou administration intramusculaire.
